# EUROPEAN PATENT APPLICATION

(11) **EP 4 566 620 A1**
(43) Date of publication of application: **11.06.2025**
(21) Application number: 23215245.4
(22) Date of filing: 08.12.2023
(51) Int. Cl.: A61K 39/00, C12N 15/113, C07K 16/28, C07K 14/725

(54) **SWITCHABLE UNIVERSAL CHIMERIC ANTIGEN RECEPTORS AND THEIR USE**

(71) Applicant: AvenCell Therapeutics Inc., Watertown, MA 02472 (US)
(72) Inventor: SPEHR, Johannes, 01307 Dresden (DE); LOFF, Simon, 01307 Dresden (DE); EHNINGER, Armin, 01307 Dresden (DE)
(74) Representative: Kailuweit & Uhlemann Patentanwälte Partnerschaft mbB

(57) **Abstract**

The present invention relates to a switchable chimeric antigen receptor (CAR) comprising a tag or tag-binding domain, which is capable of binding a tag-binding domain or a tag of a targeting module, wherein the targeting module comprises a target cell-binding domain. The invention also relates to a nucleic acid, vector or cell comprising a nucleotide sequence encoding the switchable CAR, a pharmaceutical composition comprising a cell comprising a nucleotide sequence encoding the switchable CAR, a kit and the use as a medicament, in particular for use in the treatment of cancer, infectious disease or autoimmune disease.

## Description

The present invention relates to a switchable chimeric antigen receptor (CAR) comprising a tag or tag-binding domain, which is capable of binding a tag-binding domain or a tag of a targeting module, wherein the targeting module comprises a target cell-binding domain. The invention also relates to a nucleic acid, vector or cell comprising a nucleotide sequence encoding the switchable CAR, a pharmaceutical composition comprising a cell comprising a nucleotide sequence encoding the switchable CAR, a kit and the use as a medicament, in particular for use in the treatment of cancer, infectious disease or autoimmune disease.

Chimeric antigen receptors (CARs) are artificial receptors consisting of a binding moiety, which provides the antigen-specificity, e.g., a single chain variable fragment (scFv) recognizing a surface antigen of a tumor cell, a transmembrane domain, and one or several signaling chains derived from immune receptors, e.g., CD3ζ, that activates an immune cell (Cartellieri *et al.* 2010).

The first-generation CAR comprising one signaling chain was modified by adding an intracellular signaling domain of e.g. CD28 or CD137 (4-1 BB), which are co-stimulatory molecules of a T cell, to increase the activation of immune cells (second generation CAR). A third generation CAR was developed by tandemly linking an additional complementary intracellular signaling domain derived from e.g. CD28, CD137 (4-1 BB) or CD134 (OX40), both which are tumor necrosis factor (TNF) receptor superfamily members, to a second-generation CAR. However, not all intracellular signaling domains derived from every T cell signal transducing protein sufficiently stimulate a T cell to damage and/or kill a target tumor cell. Therefore, finding intracellular signaling domains of signal transducing proteins that are effective when linked to a CAR is desirable.

Immune cells, genetically modified to express CARs, can be used to bind cells or tissue structures expressing the appropriate target of the CAR binding moiety. Cross-linking leads to an induction of signal pathways via the CAR signaling chains, which will change the biologic properties of the CAR-engrafted immune cell. In contrast, CAR activation in gene-modified regulatory T cells (Tregs) leads to an activation of Treg-specific immunomodulatory and suppressive mechanisms like interleukin (IL)-10 or tumor growth factor-beta (TGF-β) secretion. The adoptive transfer of immune cells engineered with chimeric antigen receptors (CARs) is currently considered a highly promising therapeutic option for the treatment of otherwise incurable malignant, infectious or autoimmune diseases.

However, the conventional CAR technology comes along with a number of critical issues, which need to be solved before this treatment modality can be widely applied for clinical treatments. First of all, several safety issues have to be addressed. So far, immune responses of T cells engineered with conventional CARs are difficult to control after infusion into the patient. Serious adverse event rates are high (Titov *et al.* 2018). Especially unexpected target gene expression on normal tissue may provoke a rapid and rigorous immune reaction of engineered T cells against normal cells, which can cause severe side effects (Morgan *et al*. 2010). Moreover, as CAR T cells are a new class of self-amplifying cell drugs, infused T cells can undergo a vigorous expansion in the presence of heavy tumor burden leading to tumor lysis syndrome, cytokine release syndrome and macrophage activation syndrome (Brudno and Kochenderfer 2016). Another drawback of conventional CAR technology is the restriction of engineered T cell retargeting to a single antigen. Such a monotherapeutic approach implies the risk for the development of tumor escape variants, which have lost the target antigen during treatment. The emergence of tumor escape variants under conventional CAR T cell therapy after several months was already observed in clinical trials (Sotillo *et al.* 2015). Taken together, these obstacles restrict the application of CAR T cells to very few indications. In fact, examples of clinical effectiveness have mostly been seen with CD19- and BCMA-targeting CAR T cells until now.

Modular switchable "universal" CAR T (UniCAR) approaches can overcome these limitations by separating antigen recognition and activating domain of a CAR into two separate operational units. T cells are engineered to express a CAR with a universal binding domain recognizing a tag (Cartellieri *et al*. 2016). Antigen-specificity is provided by soluble adapter molecules, which consist of an antigen-binding domain fused to the tag recognized by the UniCAR. Cartellieri *et al.* describe the treatment of CD33- and/or CD123-positive acute myeloid leukemia cells *in vitro* and *in vivo.*

Next to the UniCAR approach for recognizing various antigens (EP 2 990 416 A1), a reversed universal CAR (RevCAR) approach is known that promotes binding of an immune cell engineered to express a RevCAR comprising a tag to a target cell through an adaptor molecule comprising a tag-binding domain and a target cell binding domain (EP 3 581 200 A1).

Moreover, switchable CAR T approaches like UniCAR or RevCAR provide the possibility to rest CAR T cells in-between cycles of activation and stimulation by pausing administration of the soluble targeting module molecule. This is expected to prevent the exhaustion observed upon continuous stimulation of conventional CAR T cells thereby improving persistence (Weber *et al*. 2021).

Patel *et al.* discloses a comparison of different chimeric immune receptors comprising the same anti-HIV scFv but different extracellular spacer domains of varying size that these spacer domains significantly impact binding and function of the chimeric immune receptors (Patel *et al.* 1999). Guest *et al.* discloses that spacer size and target epitope location play a crucial role for efficiency of chimeric immune receptors directed against TAAs CD19 and CEA (Guest *et al.* 2005). Similarly, Jame *et al.* describes that the distance between receptor and target epitope must approximate the length of a TCR-pMHC complex for maximum lysis and sensitivity (Jame *et al.* 2008).

The object of the present invention is to provide a switchable chimeric antigen receptor sufficiently stimulating a T cell to damage and/or kill target cells in vivo.

According to the invention, the object is solved by the switchable chimeric antigen receptor (CAR), the nucleic acid, vector or cell comprising a nucleotide sequence encoding the switchable CAR, the pharmaceutical composition, and the kit according to the independent claims.

Advantageous embodiments of the invention are indicated in the dependent claims.

A first aspect of the invention is a switchable chimeric antigen receptor comprising
a. a tag or tag-binding domain,
b. an extracellular hinge and transmembrane domain comprising an extracellular hinge and a transmembrane domain of CD4, CD8α, CD28, ICOS (CD278), IgG1, IgG2, IgG4 or mutants and combinations thereof,
   wherein the extracellular hinge and transmembrane domain is in the range of 32 to 159 amino acids, and
c. an intracellular signaling domain that comprises at least two signal transduction domains independently selected from the group comprising a cytoplasmic region of CD3, CD27, CD28, OX40 (CD134), 4-1BB (CD137), ICOS (CD278), DAP10, DAP12, PD-1, CTLA-4, IL-2 receptor, IL-7 receptor, IL-15 receptor or IL-21 receptor and mutants thereof.

As used herein, the term "switchable chimeric antigen receptor" refers to an artificial chimeric fusion protein, in particular a receptor comprising a tag or a tag-binding domain, an extracellular hinge and a transmembrane domain and an intracellular signaling domain. The domains can be derived from different sources and therefore, the receptor is called chimeric. Advantageously, the receptor can bind with the tag or tag-binding domain, respectively, to the tag-binding domain or tag of different targeting modules, which in turn bind to an antigen on a target cell. Thus, the tag or tag-binding domain serves as target cell binding domain.

Advantageously, the switchable CAR according to the invention used in combination with a targeting module actively targets target cells, e.g. tumor cells, and is capable of inducing a significant anti-tumor response, wherein the anti-tumor response of the switchable CAR is only induced in the presence of the targeting module. The effect can be reversibly interrupted by withholding the administration of the targeting module.

The switchable chimeric antigen receptor according to the invention used in combination with a targeting module is safer and more versatile than a classical CAR construct directed against a target. The versatility of the switchable CAR platform embodies a significant advantage for treating tumors with a highly heterogeneous antigenic profile. This therapy can be quickly adapted to the evolving antigenic profile to avoid a treatment-induced selection of tumor cells lacking the targeted antigen and the consequent tumor relapse. Moreover, only one switchable CAR-T cell product is required for different indications and targets.

Advantageously, the length of the switchable CAR according to the invention, in particular the length of the hinge and transmembrane domain, was adapted resulting in an increased efficiency of the target cell binding and/or lysis.

As used herein, the term "domain" refers to a part of a protein sequence, which can exist and function independently from the rest of the protein.

As used herein, the term "targeting module" refers to a molecule, preferably a polypeptide or protein, with at least two different domains, wherein each domain is specific for a target or a uniform group of targets, respectively, wherein at least one domain is specific for a target cell, e.g., a CD123-binding domain; and one domain is specific for a switchable CAR, in particular the tag or tag-binding domain of the switchable CAR.

As used herein, the term "target cell-binding domain" refers to a protein, peptide, or low molecular weight organic ligand, which specifically binds a protein or protein complex (antigen) on the surface of a target cell, preferably a cancer cell, B cell, T cell, plasma cell, infected cell, pathogen or parasite.

As used herein, the term "specific" refers to the ability of an antibody, antibody fragment or a protein, peptide or low molecular weight organic ligand to recognize and bind with a binding partner (e.g., a tumor antigen) protein present in a sample, but not substantially recognize or bind other molecules in the sample.

As used herein, the term "binds" or "binding" refers to a non-covalent binding, in particular ionic bonds, hydrogen bonds, Van der Waals forces and/or hydrophobic interactions.

As used herein, the terms "peptide", "polypeptide" and "protein" are used interchangeably and refer to a compound comprised of amino acid residues covalently linked by peptide bonds. A protein or peptide must contain at least two amino acids, and no limitation is placed on the maximum number of amino acids that can comprise a proteins or peptides sequence.

As used herein, the term "low molecular weight organic ligand" refers to an organic molecule with a molecular weight of maximal 10 kilodaltons, preferably of maximal 3 kilodaltons, which specifically binds a protein or protein complex (antigen) on the surface of a target cell, preferably a cancer cell, B cell, T cell, plasma cell, infected cell or pathogen or parasite.

As used herein, the term "mutants" refers to peptides or proteins having at least 90% sequence identity to the named antibodies, antibody fragments, proteins or peptides, preferably at least 95 %, more preferably at least 99%, sequence identity. Advantageously, the mutants are capable of having one or more activities of the named domains, antibodies, antibody fragments, peptides or proteins.

In embodiments, the mutant comprises a point mutation. As used herein, a "point mutation" is a mutation, wherein a single nucleotide base is changed, inserted or deleted from nucleotide sequence. In embodiments, mutants are truncated variants of peptides or proteins. As used herein, the term "truncated versions" refers to shortened peptides or proteins having at least 90% sequence identity to the named peptides or proteins, preferably at least 95 %, more preferably at least 99%, sequence identity, more preferably having a chain length of at least 90 % and a sequence identity of 100 %, most preferably a chain length of at least 95 % and a sequence identity of 100 %, resulting from a mutation in the nucleotide sequence coding for the peptide or protein. Advantageously, the truncated version has at least 80 %, preferably of at least 90 %, more preferably of at least 95 %; of the activity of the named peptide or protein.

In embodiments, the switchable CAR comprises a tag. As used herein, the term "tag" refers to a marker, in particular a peptide sequence or an organic molecule, attached to peptides or proteins to enable them to bind to specific atoms, ions or molecules, in particular the tag-binding domain.

In embodiments, the tag-binding domain or tag is present at the amino-terminal end of the polypeptide that comprises the switchable CAR. Advantageously, locating the tag-binding domain or the tag at the amino terminus permits unhampered access to the targeting module that is bound to the target cell.

In embodiments, the tag is selected from organic molecules including fluorescence labels, e.g., FITC (Fluorescein isothiocyanate), and biotin.

In embodiments, the tag is a peptide epitope tag. In further embodiments, the tag comprises 10 to 20 amino acids.

In embodiments, the peptide epitope tag is a myc-tag, a His-tag, a peptide sequence from yeast transcription factor GCN4, preferably according to SEQ ID No. 1, SEQ ID No. 2 or mutants thereof; a leucine zipper sequence, preferably SYNZIP 1 to SYNZIP 48, BATF, FOS, ATF4, ATF3, BACH1, JUND, NFE2L3, HEPTAD (Reinke *et al.* 2010), a sequence according to SEQ ID No. 3 or SEQ ID No. 4 or mutants thereof; or a peptide sequence from a human protein, preferably from a human nuclear protein, more preferably from the human La protein, even more preferably according to SEQ ID No. 5, SEQ ID No. 6, SEQ ID No. 7 or mutants thereof.

As used herein, the term "nuclear protein" refers to a protein found in the cell nucleus. Advantageously, tags, which are peptide sequences from nuclear antigens, cannot be accessed and bound by the corresponding tag-binding domain in the context of the native protein under physiological conditions. This leads to minimization of the risk of uncontrolled on-target off-site toxicities by CAR-expressing immune cells like the release of toxic levels of cytokines, referred to variously as cytokine storms or cytokine release syndrome (CRS). Further advantageously, the tag is not immunogenic.

In embodiments, the human protein is a human Alpha-fetoprotein or a human nuclear protein, more preferably from the human La protein.

In embodiments, the His-tag is an amino acid sequence consisting of histidine residues, preferably in the range of six to fourteen histidine residues.

In preferred embodiments, the peptide epitope tag is a myc-tag, a His-tag, a peptide sequence from yeast transcription factor GCN4, preferably according to SEQ ID No. 1 or SEQ ID No. 2; a leucine zipper sequence, preferably SYNZIP 1 to SYNZIP 48, BATF, FOS, ATF4, ATF3, BACH1, JUND, NFE2L3, HEPTAD, a sequence according to SEQ ID No. 3 or SEQ ID No. 4; or a peptide sequence from a human nuclear protein, preferably from the human La protein according to SEQ ID No. 5, SEQ ID No. 6 or SEQ ID No. 7.

Preferably, the peptide epitope tag from the human La protein is the human La epitope E5B9 according to SEQ ID No. 5 or E7B6 according to SEQ ID No. 6 or SEQ ID No. 7, most preferably the human La epitope E5B9 according to SEQ ID No. 5 or E7B6 according to SEQ ID No. 7.

In embodiments, the switchable CAR comprises a tag-binding domain.

In embodiments, the tag-binding domain is an antibody or antigen-binding fragment.

In embodiments, the tag-binding domain is an antibody or an antigen-binding fragment binding to a myc-tag, a His-tag, a peptide sequence from yeast transcription factor GCN4, preferably according to SEQ ID No. 1, SEQ ID No. 2 or mutants thereof; a leucine zipper sequence, preferably SYNZIP 1 to SYNZIP48, BATF, FOS, ATF4, ATF3, BACH1, JUND, NFE2L3, HEPTAD (Reinke *et al.* 2010), a sequence according to SEQ ID No. 3 or SEQ ID No. 4 or mutants thereof; or a peptide sequence from a human protein. In embodiments, the tag-binding domain is an antibody or an antigen-binding fragment binding to a myc-tag, a His-tag, a peptide sequence from yeast transcription factor GCN4 according to SEQ ID No. 1, SEQ ID No. 2, a leucine zipper sequence selected from SYNZIP 1 to SYNZIP 48, BATF, FOS, ATF4, ATF3, BACH1, JUND, NFE2L3, HEPTAD (Reinke *et al.* 2010), a sequence according to SEQ ID No. 3 or SEQ ID No. 4 or a peptide sequence from a human nuclear protein.

As used herein, the term "antibody" refers to a protein, which binds antigens via the antigen-binding fragment variable region (Fab). This is composed of one constant and one variable domain of each of the heavy (V_{H}) and the light chain (V_{L}). As used herein, the term "antibody fragment" or "antigen-binding fragment" refers to a protein comprising at least the V_{L} or V_{H} of an antibody. In embodiments, antibody fragments are selected from single-chain variable fragments (scFv), single-chain antibodies, Fc fragment, F(ab')2 fragments, Fab fragments, and fragments produced by a Fab expression library or single-domain antibodies (nanobodies).

As used herein, the term "Fab fragments" refers to an antibody fragment comprising one constant and one variable domain of each of the heavy and the light chain.

As used herein, the term "single-chain variable fragment (scFv)" refers to an artificial antibody fragment comprising a variable domain of a light chain and a variable domain of a heavy chain of an antibody covalently linked. In embodiments, the V_{L} and V_{H} of an antibody are covalently linked by a short peptide of 10 to 25 amino acids. In further embodiments, the short peptide links the N-terminus of the V_{H} with the C-terminus of the V_{L}, or vice versa.

As used herein, the term "single-domain antibody (sdAb)" or "nanobody" is an antibody fragment consisting of a single monomeric variable antibody domain, in particular a VHH fragment.

In embodiments, the antibody is obtained from an animal species, preferably from a mammal such as human, simian, mouse, rat, rabbit, lama, alpaca, camel, guinea pig, horse, cow, sheep, goat, pig, dog or cat. In embodiments, the antibody is derived from a shark. Preferably, the antibody or antibody fragment is a human, humanized or deimmunized antibody. Humanized antibodies can be prepared in various ways, for example, by resurfacing and CDR grafting. In case of resurfacing, a combination of molecular modeling, statistical analyses, and mutagenesis is used to modify all non-CDR regions on the surface of the antibody to become similar to the surface of antibodies of the target organism. In CDR grafting, the CDR regions according to the invention are introduced into known human framework regions, which are similar in sequence to the original ones. Deimmunized antibodies can be obtained by specifically mutating residues that confer immunogenicity hotspots as predicted based on in silico peptide-MHC affinity prediction.

In embodiments, the antibody or antibody fragment is a polyclonal, a monoclonal or a chimeric antibody, wherein an antigen-binding region of a non-human antibody is transferred into the framework of a human antibody by recombinant DNA techniques including in silico design.

In embodiments, antibodies to a selected tag or antigen may be produced by immunization of various hosts including, but not limited to, goats, rabbits, rats, mice, humans, through injection with cells expressing a particular protein, DNA or RNA encoding for the protein, the protein itself or any portion, fragment or oligopeptide that retain immunogenic properties of the protein.

In embodiments, the antibody, antigen-binding fragment, protein or peptide comprises modifications selected from the group comprising D amino acids, pseudo peptide bonds, aminoalcohols, non-proteinogenic amino acids, unnatural amino acids, amino acids with modified side chains and/or circular proteins. Advantageously, these analogues reveal increased stability.

In embodiments, the variable region(s) of the at least one tag-binding domain comprise(s) a humanized amino acid sequence.

In embodiments, the tag-binding domain is an antigen-binding fragment. In embodiments, the tag-binding domain is an scFv or a Fab fragment.

In embodiments, the tag-binding domain is an scFv binding a La epitope. In embodiments, the tag-binding domain is an scFv binding La epitope 5B9 or 7B6 according to SEQ ID No. 5, SEQ ID No. 6 or SEQ ID No. 7.

In embodiments, V_{L} and V_{H} are connected via a glycine-serine linker with the structure (GₓS_{y}) with x and y selected from 1 to 10, preferably 3 to 5. Mostly preferred are 1 to 10 repeats of the sequence G₄S₁ (SEQ ID No. 20). In embodiments, linkers are used that are constituted of a peptide sequence that can increase the protease resistance of the antibody derivatives. As used herein, the term "linker" (also spacer) refers to a molecule or molecule part separating at least two elements under consideration, in particular selected from functional groups, tags, binding domains or binding domain subunits, such as a V_{L} and a V_{H} domain.

In embodiments, the linker comprises 20 to 30 amino acids, preferably 25 amino acids.

In embodiments, the linker is an amino acid sequence according SEQ ID No. 21 or SEQ ID No. 22.

As used herein, the term "CDR (Complementarity-determining regions)" refers to parts of the variable chains in antibodies or antibody fragments, where the antibodies or antibody fragments bind to their specific antigen. An antibody comprises three CDRs (CDR1, CDR2 and CDR3), arranged non-consecutively, on the amino acid sequence of each variable domain and thus, six CDRs on the two variable domains (V_{H} and V_{L}), which can come into contact with the antigen.

In embodiments, the tag-binding domain binding a human La epitope E5B9 comprises CDR sequences according to SEQ ID No. 8, SEQ ID No. 9, SEQ ID No. 10, SEQ ID No. 11, amino acid sequence WAS (Trp-Ala-Ser) and SEQ ID No. 12.

In embodiments, the tag-binding domain is an antibody or an antigen-binding fragment comprising a V_{L} according to the following sequence:
DIVMTQSPDSLAVSLGERATINCX₂₄SSQSLLNSRTX₃₅KNYLAWYQQKPGQPPKLLIYWASTR X₆₁SGVPDRFSGSGSGTDFTLTISSLQAEDVAVYYCKQSYNLX₁₀₁TFGGGTKVEIK (SEQ ID No. 13), wherein X₂₄, X₃₅, X₆₁ and X₁₀₁ are independently from each other selected from a proteinogenic alpha-amino acid residue; or an amino acid sequence having at least 90 % sequence identity, preferably at least 95% sequence identity; to sequence SEQ ID No. 14 or SEQ ID No. 16.

In some embodiments, X₂₄ to X₁₀₁ are selected as follows:
X₂₄ is selected from polar and/or positive charged residues, such as Serine, Threonine, Asparagine, Glutamine, Histidine, Lysine and Arginine; preferably Lysine or Arginine;
X₃₅ is preferably selected from Lysine and Proline;
X₆₁ is selected from polar and charged residues, such as Asparagine, Aspartic Acid, Glutamine, Glutamic acid, Histidine, Lysine and Arginine, preferably Glutamic acid and Lysine;
X₁₀₁ is selected from hydrophobic residues, such as Isoleucine, Leucine, Valine, Alanine, Methionine, Phenylalanine, Proline and Tryptophan; preferably Leucine or Proline.

In embodiments, the tag-binding domain is an antibody or an antigen-binding fragment comprising a V_{H} with an amino acid sequence having at least 90 % sequence identity, preferably at least 95 % sequence identity; to sequence SEQ ID No. 15 or SEQ ID No. 17.

In embodiments, the tag-binding domain comprises a sequence having each at least 90 % sequence identity, preferably at least 95 % sequence identity; to the sequences according to SEQ ID No. 14 (V_{L}) and SEQ ID No. 15 (V_{H}).

In embodiments, the tag-binding domain is an anti-La 5B9 scFv comprising SEQ ID No. 14 (V_{L}) and SEQ ID No. 15 (V_{H}).

In embodiments, the tag-binding domain comprises a sequence having each at least 90 % sequence identity, preferably at least 95 % sequence identity; to the sequences according to SEQ ID No. 16 (V_{L}) and SEQ ID No. 17 (V_{H}).

In embodiments, the tag-binding domain is an anti-La 7B6 scFv comprising SEQ ID No. 16 (V_{L}) and SEQ ID No. 17 (V_{H}).

In embodiments, the tag-binding domain comprises a V_{L}-linker-V_{H} structure, wherein the V_{L} region of the tag-binding domain comprises a sequence with at least 95 % identity, preferably 99 % identity, with the sequence according to SEQ ID No. 14 and/or the V_{H} region of the tag-binding domain comprises a sequence with at least 95 % identity, preferably 99 % identity, with the sequence according to SEQ ID No. 15. As used herein, the term "V_{L}-linker-V_{H} structure" refers to a structure, wherein the C-terminus of the V_{L} region is connected with a linker, which is connected to the N-terminus of the V_{H} region.

In embodiments, the tag-binding domain is a Fab fragment binding a La epitope. In embodiments, the tag-binding domain is a Fab fragment binding La epitope 5B9 comprising an amino acid sequence according to SEQ ID No. 18 and SEQ ID No. 19.

As used herein, the term "extracellular hinge and transmembrane domain" refers to a flexible peptide sequence connected to the tag or tag-binding domain, which anchors the switchable CAR into the cell membrane of the cell and protrudes from the surface of the cell for optimal binding to its particular targeting module.

According to the invention, the extracellular hinge and transmembrane domain comprises an extracellular hinge and a transmembrane domain of CD4, CD8α, CD28, ICOS (CD278), IgG1, IgG2, IgG4 or mutants and combinations thereof, wherein the extracellular hinge and transmembrane domain is in the range of 32 to 159 amino acids, preferably in the range of 32 to 88 amino acids, more preferably in the range of 32 to 64 amino acids.

As used herein, the term "extracellular hinge domain" refers to a peptide sequence between the transmembrane domain and the tag or tag-binding domain and which protrudes from the surface of the cell for optimal binding to its particular targeting module. Advantageously, the extracellular hinge domain enables different alignments of the CAR and thus the positioning of the CAR (effector cell) to the targeting module, affecting the binding of the targeting module by the CAR (effector cell). Despite the shortening of the extracellular domain, an increase in efficiency was observed. Further advantageously, the extracellular hinge domain enables binding of various targeting module formats directed against various target epitopes and efficient immune synapse formation. Switchable CARs with shortened extracellular domain showed an increase in efficiency in combination with various targeting module formats and against various target epitopes.

In embodiments, the switchable CAR comprises an extracellular hinge domain of human CD4, CD8α, CD28, ICOS (CD278), IgG1, IgG2 or IgG4 or mutants thereof, wherein the extracellular hinge domain is in the range of 12 to 133 amino acids, preferably in the range of 12 to 62 amino acids, more preferably in the range of 12 to 38 amino acids.

In embodiments, the switchable CAR comprises an extracellular hinge domain of human CD8α, CD28, IgG4 or mutants thereof. In embodiments, the switchable chimeric antigen receptor comprises an extracellular hinge domain of human CD8α according SEQ ID No. 23 or SEQ ID No. 24, CD28 according SEQ ID No. 25 or SEQ ID No. 26 or IgG4 according SEQ ID No. 27.

In embodiments, the switchable chimeric antigen receptor comprises an extracellular hinge domain of human CD8α according SEQ ID No. 23 or SEQ ID No. 24, CD28 according SEQ ID No. 25 or IgG4 according SEQ ID No. 27. In embodiments, the switchable chimeric antigen receptor comprises an extracellular hinge domain of human IgG4 according SEQ ID No. 27.

As used herein, the term "transmembrane domain" refers to a membrane-spanning protein domain or the part of the extracellular hinge and transmembrane domain, which anchors the switchable CAR into the cell membrane of the cell. In embodiments, a transmembrane domain consists of at least one alpha-helix or a transmembrane beta barrel.

In embodiments, the switchable CAR comprises a transmembrane domain of human CD8α, CD28, ICOS (CD278) or mutants thereof, wherein the transmembrane domain is in the range of 20 to 26 amino acids.

In embodiments, the switchable CAR comprises a transmembrane domain of human CD8α according SEQ ID No. 28 or CD28 according SEQ ID No. 29.

In embodiments, combinations of the extracellular hinge and transmembrane domain are CD8α extracellular hinge and transmembrane domain, CD28 extracellular hinge and transmembrane domain, a CD28 extracellular hinge domain combined with a CD8α transmembrane domain, an IgG4 extracellular hinge domain combined with a CD8α transmembrane domain or an IgG4 extracellular hinge domain combined with a CD28 transmembrane domain. In embodiments, the switchable CAR comprises an extracellular hinge and transmembrane domain according to one of the amino acid sequences SEQ ID No. 30 to SEQ ID No. 35. In preferred embodiments, the switchable CAR comprises an extracellular hinge and transmembrane domain according to one of the amino acid sequences SEQ ID No. 33 to SEQ ID No. 35.

In embodiments, the extracellular hinge domain and/or the transmembrane domain comprises additional 1 to 25 flanking amino acids derived from the source protein.

In embodiments, the extracellular hinge domain and/or the transmembrane domain comprises a linker sequence of preferably 1 to 25 amino acid residues.

Preferred linkers are glycine-serine linkers with the structure (GₓS_{y}) with x and y selected from 1 to 10, preferably 1 to 5. Mostly preferred are 1 to 10 repeats of the sequence G₄S₁ (SEQ ID No. 20). Moreover, linkers are preferred that are constituted of a peptide sequence that can increase the protease resistance of the antibody derivatives.

In embodiments, the linker is SEQ ID No. 21 or SEQ ID No. 22.

In embodiments the linkers comprise 3-24 alanine residues, preferably multiples of 3.

In embodiments, the mutant of the extracellular hinge domain and/or the transmembrane domain comprises one- or two-point mutations. In embodiments, the mutants are truncated variants of the extracellular hinge domain and/or the transmembrane domain, domains having a chain length of at least 90 % and a sequence identity of 100 %, most preferably a chain length of at least 95 % and a sequence identity of 100 %, resulting from a mutation in the nucleotide sequence coding for the domain.

According to the invention, the intracellular signaling domain comprises at least two signal transduction domains independently selected from a cytoplasmic region of a CD3, CD27, CD28, CD134 (OX40), CD137 (4-1BB), CD278 (ICOS), DAP10, DAP12, programmed cell death-1 (PD-1), cytotoxic T-lymphocyte antigen 4 (CTLA-4), IL-2 receptor, preferably CD122 (interleukin-2 receptor β) or CD132 (interleukin-2 receptor γ); IL-7 receptor, preferably CD127 (interleukin-7 receptor α); IL-15 receptor or CD360 (interleukin-21 receptor), and mutants thereof.

As used herein, the term "intracellular signaling domain" refers to a peptide sequence which transmits a signal into the cell by cross-linkage of the cell expressing the switchable CAR (effector cell) to a human cell surface protein or protein complex (target cell). Cross-linkage between effector and target cell is mediated by the targeting module.

As used herein, the term "mutants" refers to proteins having at least 90 % sequence identity to the signal transduction domains, preferably at least 95 % sequence identity. Advantageously, the mutant transmits a signal into the cell by cross-linkage of the cell expressing the switchable CAR (effector cell) to a human cell surface protein or protein complex (target cell) in the same way as the named signal transduction domains.

In embodiments, mutants are truncated versions. As used herein, the term "truncated versions" refers to shortened proteins having at least 90 % sequence identity to the signal transduction domains, preferably at least 95 % sequence identity, more preferably having a chain length of at least 90 % and a sequence identity of 100 %, most preferably a chain length of at least 95 % and a sequence identity of 100 %. Advantageously, the truncated version has an activity of at least 80 %, preferably of at least 90 %, more preferably of at least 95 %; of the named signal transduction domains.

Hombach *et al.* describes the use of cytoplasmic regions of CD28 as signal transduction domain in CARs (Hombach *et al.* 2001). Guedan *et al.* describes the use of a mutant of cytoplasmic regions of CD28 as signal transduction domain (Guedan *et al.* 2020).

Finney *et al.* describes the use of cytoplasmic regions of CD137 (4-1BB) and of CD134 (OX40) as signal transduction domain (Finney *et al.* 2004).

Guedan *et al.* describes the use of cytoplasmic regions of CD278 (ICOS) as signal transduction domain (Guedan *et al.* 2018).

Zhang *et al.* describes the use of DAP10 as signal transduction domain (Zhang *et al.* 2005).

Fedorov *et al*. describes the use of programmed cell death 1 (PD-1) and of cytotoxic T-lymphocyte antigen 4 (CTLA-4) as signal transduction domain in CARs (Fedorov *et al.* 2013).

Gong *et al.* describes the use of cytoplasmic regions of CD3 chains, in particular the CD3ζ chain, as signal transduction domain in CARs (Gong *et al.* 1999).

Töpfer *et al.* describes the use of DAP12 as signal transduction domain in CARs (Töpfer *et al.* 2015).

Kagoya *et al.* describes the use of signaling chains or motifs derived from interleukin receptors as signal transduction domain in CARs (Kagoya *et al.* 2018).

In embodiments, the intracellular signaling domain comprises at least a cytoplasmic region of CD3ζ, 4-1BB, CD28 or mutants thereof.

In embodiments, the intracellular signaling domain comprises two or three signal transduction domains selected from the group comprising CD28, 4-1BB, ICOS, CD3ζ, IL-7Rα and mutants thereof.

In embodiments, the intracellular signaling domain comprises at least a cytoplasmic region of CD28 or mutants thereof, preferably an amino acid sequence according to SEQ ID No. 37 or SEQ ID No. 38.

In embodiments, the intracellular signaling domain comprises at least a cytoplasmic region of 4-1BB or mutants thereof, preferably an amino acid sequence according to one of the sequences SEQ ID No. 39 to SEQ ID No. 40.

In further embodiments, the intracellular signaling domain comprises at least a cytoplasmic region of CD3ζ or mutants thereof, preferably according to SEQ ID No. 41.

In further embodiments, the switchable CAR comprises a further domain, wherein the further domain is a short peptide linker in the extracellular portion of the receptor that may serve to detect the chimeric antigen receptor on the cell surface or stimulate the chimeric antigen receptor T cell. Advantageously, the switchable CAR engrafted cells with the further domain can be specifically stimulated to proliferate preferentially and persist longer compared to non-engrafted cells either *in vitro* or *in vivo.* Further advantageously, the further domain may also be used to purify switchable CAR engrafted cells from mixed cell populations or to dampen switchable CAR engrafted cell-mediated immune response and to eliminate switchable CAR engrafted cells *in vivo.*

In embodiments, the further domain forms a linear epitope for a monoclonal antibody (mab) specifically binding to the further domain. In some embodiments, the further domain comprises at least one linear epitope, preferably E7B6 according to SEQ ID No. 6 or SEQ ID No. 7.

In embodiments, the further domain is located in between the tag or tag-binding domain and the extracellular hinge domain or is an integral part of the extracellular hinge domain.

In embodiments, the switchable CAR is a reversible chimeric antigen receptor (RevCAR) comprising a tag. In embodiments, the RevCAR comprises a tag present at the amino-terminal end.

In embodiments, the switchable CAR comprises a signal peptide selected from a human CD8α, CSF2Rα, CD3ζ, IL-2, lysozyme C, a heavy chain of an antibody, a light chain of an antibody or a part of a light chain of an antibody or mutants thereof. In embodiments, the signal peptide is an amino acid sequence selected from the group comprising SEQ ID No. 42 to SEQ ID No. 46. Suitably, the signal peptide is located at the N-terminus of the switchable CAR sequence ahead of the tag, the extracellular hinge and transmembrane domain and the intracellular signaling domain.

As used herein, the term "signal peptide" (also leader peptide) refers to a short amino acid sequence at the N-terminus of proteins meant for secretion or membrane localization. Advantageously, the signal peptide effects the transport of the switchable CAR on the cell surface of an effector cell.

In embodiments, the switchable universal chimeric antigen receptor comprises a signal peptide of a human immunoglobulin with an amino acid sequence in the range of 16 to 30 amino acids
The term "immunoglobulin" refers to Y-shaped protein consisting of two identical heavy chains and two identical light chains connected by disulfide bonds used by the immune system to identify and neutralize foreign objects such as pathogenic bacteria and viruses, called antigens. Advantageously, using signal peptides influences the switchable CAR expression levels on the surface of an effector cell and thus, the efficiency.

In embodiments, the switchable CAR comprises a sequence according to SEQ ID No. 53 to SEQ ID No. 112. In embodiments, the switchable CAR comprises a sequence according to SEQ ID No. 53 to SEQ ID No. 82.

Another aspect of the invention is a nucleic acid, vector or cell comprising a nucleotide sequence encoding the switchable CAR according to the invention. The nucleic acid, vector and/or cell are isolated.

In embodiments, the nucleic acid, vector or cell comprises a nucleotide sequence encoding a signal peptide according to SEQ ID No. 47 to SEQ ID No. 51.

In embodiments, the nucleotide sequence encoding a switchable chimeric antigen receptor comprises a nucleotide sequence according to SEQ ID No. 113 to SEQ ID No. 142. The nucleic acid, vector and/or cell are isolated.

In embodiments, the vector is a plasmid, an artificial chromosome, linearized DNA or RNA, a virus particle or another vector that contains an expression cassette that is incorporated stably into the genome of a host cell or host organism.

In embodiments, the vector further comprises a promoter, wherein the promoter is selected from the group comprising an EF-1 promoter, a CMV IE gene promoter, an EF-1α promoter, a ubiquitin C promoter, or a phosphoglycerate kinase (PGK) promoter.

In embodiments, the cell comprises an exogenous nucleotide sequence encoding the switchable CAR that is expressed on the surface of the cell.

In embodiments, the cell is selected from immune cells, preferably with cytolytic, phagocytic or immunosuppressive activity, such as T cells, Natural Killer (NK) cells and macrophages. In embodiments, the cell is selected from T cells, including alpha/beta and gamma/delta T cells or subpopulations of T cells like stem-cell memory T cells or central memory T cells, cytotoxic T cells or NK cells.

In embodiments, the vector or cell further comprises an inducible expression system. In some embodiments, the inducible expression system is based on a prokaryotic operon, including, but not limited to, the lac operon, transposon Tn₁₀ or tetracycline operon. In other embodiments, the inducible expression system is based on components of a eukaryotic signaling pathway, including, but not limited to, expression systems based on a steroid receptor, an estrogen receptor, progesterone or metallothionein.

In embodiments, the inducible expression system induces the transcription of the nucleotide sequence encoding a switchable CAR and optionally a nucleotide sequence encoding a targeting module.

In embodiments, the cell is an engineered human cell comprising at least one genetic modification.

In embodiments, the cell is an engineered allogeneic immune cell. In embodiments, the engineered allogeneic immune cell is obtained by at least one genetic modification of a donor cell.

In embodiments, the cell comprises a reduced or eliminated surface expression of endogenous T cell receptor alpha chain (TRAC) by a genetic modification in the T cell receptoralpha chain gene, a reduced or eliminated surface expression of HLA-A relative to an unmodified T cell by a genetic modification in the HLA-A gene, and/or a reduced or eliminated surface expression of HLA class II by a genetic modification in the CIITA gene. Advantageously, the three knock outs (TRAC, HLA-A gene, CIITA) result in a reduction of the rejection by the recipient subject's immune cells, in particular decrease the chance of GvHD for allogeneic T cells.

In embodiments, the genetic modification in the TRAC gene comprises at least one nucleotide within the genomic coordinates chr14:22547524-chr14:22547544. In embodiments, the genetic modification in the TRAC gene comprises at least 10 or at least 15 contiguous nucleotides within the genomic coordinates.

In embodiments, the genetic modification in the TRAC gene comprises at least one nucleotide of an exon of the TRAC gene.

In embodiments, the genetic modification in the TRAC gene comprises at least one insertion, deletion, substitution, or deamination of at least one nucleotide within the genomic coordinates.

In embodiments, the genetic modification in the TRAC gene comprises an indel.

In embodiments, the genetic modification in the HLA-A gene comprises at least one nucleotide within the genomic coordinates chosen from: chr6:29942854-chr6:29942913 and chr6:29943518-chr6:29943619.

In embodiments, the T cell is homozygous for HLA-B and/or homozygous for HLA-C genotypes. Preferably, the T cell is homozygous for HLA-B and HLA-C genotypes.

In embodiments, the T cell has reduced or eliminated expression of at least one HLA-A allele selected from: HLA-A1, HLA-A2, HLA-A3, HLA-A11, and HLA-A24.

In embodiments, the genetic modification in the HLA-A gene comprises at least 5, 6, 7, 8, 9, or 10 contiguous nucleotides within the genomic coordinates, preferably at least 10, at least 15, at least 16, at least 17, at least 18, at least 19, or at least 20 contiguous nucleotides within the genomic coordinates.

In embodiments, the genetic modification in the HLA-A gene comprises at least one C to T substitution or at least one A to G substitution within the genomic coordinates.

In embodiments, the genetic modification in the HLA-A gene comprises an indel.

In embodiments, the T-cell further comprises a reduced or eliminated surface expression of HLA-B relative to an unmodified T cell by a genetic modification in the HLA-B gene and/or a reduced or eliminated surface expression of HLA-C relative to an unmodified T cell by a genetic modification in the HLA-C gene.

In further embodiments, the T-cell further comprises a reduced or eliminated surface expression of HLA-B relative to an unmodified T cell by a genetic modification in the HLA-B gene and the T cell is homozygous for HLA-C genotype.

In alternative embodiments, the T-cell further comprises a reduced or eliminated surface expression of HLA-C relative to an unmodified T cell by a genetic modification in the HLA-C gene and the T cell is homozygous for HLA-B genotype.

In further alternative embodiments, the T-cell further comprises a reduced or eliminated surface expression of HLA-B relative to an unmodified T cell by a genetic modification in the HLA-B gene and a reduced or eliminated surface expression of HLA-C relative to an unmodified T cell by a genetic modification in the HLA-C gene.

In embodiments, the genetic modification in the CIITA gene comprises at least one nucleotide of at least one nucleotide of a splice site within the genomic coordinates chr16:10902171-chr16:10923242.

In embodiments, the genetic modification in the CIITA gene comprises a modification of at least one nucleotide of a splice acceptor site, preferably wherein the one nucleotide is A or G or T.

In embodiments, the genetic modification in the CIITA gene comprises a modification of a splice site boundary nucleotide.

In embodiments, the genetic modification in the CIITA gene comprises at least 5, 6, 7, 8, 9, or 10 contiguous nucleotides within the genomic coordinates chr16:10902171- chr16:10923242.

In embodiments, the genetic modification in the CIITA gene comprises at least 10 or at least 15 contiguous nucleotides within the genomic coordinates.

In embodiments, the genetic modification in the CIITA gene comprises an indel.

A further aspect of the invention is a pharmaceutical composition comprising a cell comprising a nucleotide sequence encoding the switchable CAR according to the invention and a pharmaceutically acceptable thinner or carrier.

In embodiments, the pharmaceutical composition is administered parenterally, particularly preferred intravenously. In embodiments, the pharmaceutical composition is present in a form suitable for intravenous administration. Preferably, the pharmaceutical composition is a solution, emulsion or suspension.

In embodiments, the pharmaceutical composition is an injectable buffered solution comprising a concentration in the range of 1·10⁵ to 1·10⁸ per mL of the cell comprising a nucleotide sequence encoding the switchable CAR according to the invention.

The pharmaceutical composition comprises a pharmaceutically acceptable thinner (dilution agent) or carrier. In embodiments, the carrier is selected from water, an aqueous buffer solution, 0.9 % saline solution, 5 % glucose, 5 % xylitol, 0.3 % glycine solution, ringer solutions or amino acid solutions. In further embodiments, the aqueous buffer solution is selected from an aqueous histidine, sodium succinate, sodium citrate, sodium phosphate or potassium phosphate-buffered solution with a pH value in the range of pH 5.0 to pH 7.0. In embodiments, the aqueous buffer solution has a buffer concentration in the range of 1 mmol/l (mM) to 500 mM, preferably in the range of 5 mM to 20 mM, more preferably in the range of 5 mM to 10 mM.

In embodiments, the carrier comprises sodium chloride. In embodiments, the carrier comprises sodium chloride with a concentration in the range of 1 mM to 300 mM. In embodiments, the carrier comprises sodium chloride with a concentration of about 150 mM.

In embodiments, the pharmaceutical composition comprises a stabilizer. In embodiments, the pharmaceutical composition comprises a stabilizer with a concentration in the range of 1 mM to 900 mM. In embodiments, the pharmaceutical composition comprises a stabifzer with a concentration in the range of 50 mM and 600 mM. In embodiments, the stabilizer is sucrose, trehalose or L-methionine.

In embodiments, the pharmaceutical composition further comprises pharmaceutically acceptable excipients. The term "pharmaceutically acceptable excipients" refers to compounds, which provide approximately physiological conditions and/or increase the stability, such as agents for adjusting the pH value and buffering agents, agents for adjusting the toxicity and the like. In embodiments, pharmaceutically acceptable excipients are selected from sodium acetate, sodium chloride, potassium chloride, calcium chloride, sodium lactate and polysorbate-80, preferably polysorbate-80 in the range of 0.0001 % (w/v) to 1 % (w/v). In embodiments, the concentration of the pharmaceutically acceptable excipient is in the range of 0.001 % (w/v) to 0.1 % (w/v).

In embodiments, the pharmaceutical composition further comprises a targeting module.

In embodiments, the pharmaceutical composition comprises at least one targeting module, wherein the at least one targeting module comprises at least one target cell-binding domain and a tag or a tag-binding domain, wherein the at least one target cell-binding domain is an antibody, antibody fragment, a protein, a peptide or a low molecular weight organic ligand that binds to surface antigens selected from the group comprising CD2, CD3, CD4, CD5, CD7, CD8, CD10, CD15, CD19, CD20, CD22, CD23, CD25, CD30, CD33, CD38, CD44, CD44v6, CD52, CD66a, CD66b, CD66c, CD66d, CD66e, CD66f, CD79a, CD79b, CD90, CD99, CD123, CD133, CD135, CD150, CD181, CD182, CD184, CD223, CD229, CD269, CD273, CD274, CD276, CD279, CD319, CD366, CD371, a cytokine receptor, CXCR4, c-Met, mesothelin, a member of the epidermal growth factor receptor family or a mutant thereof, a member of the tumor necrosis factor receptor superfamily, a claudin, an ephrin, an ephrin receptor, a fucosyl transferase, a prostate specific antigen, an embryonic antigen, a member of the vascular endothelia growth factor family, an epithelial cell adhesion molecule, an alpha-fetoprotein, a member of the intercellular adhesion molecule family, a C-type lectin, an integrin, a member of the mucin protein family, a follicle-stimulating hormone receptor, a high molecular weight-melanoma associated antigen, a folate binding protein, a folate receptor, a somatostatin receptor, a ligand of the NKG2D receptor, a member of the epithelia glycoprotein family, a disialoganglioside, a glypican, a G protein-coupled receptor, a human papillomavirus protein, cancer/testis antigen, fibroblast activation protein, a member of the carbonic anhydrase family, a member of the carbohydrate antigen family, a Notch ligand, melanoma-associated chondroitin sulfate proteoglycan, glycoprotein A33, guanylate cyclase 2C and a tumor-specific glycan.

In embodiments, the pharmaceutical composition is an injectable buffered solution comprising a concentration in the range of 1·10⁵ to 1·10⁸ per mL of the cell comprising a nucleotide sequence encoding the switchable CAR according to the invention and a concentration in the range of 1 ng/ml to 500 mg/ml of the targeting module. In embodiments, the pharmaceutical composition comprises a concentration in the range of 50 µg/ml to 5 mg/ml of the targeting module.

In embodiments, the pharmaceutical composition comprises the targeting module in a dosage quantity in the range of 25 µg/day to 100 mg/day. In embodiments, the pharmaceutical composition comprises the targeting module in a dosage quantity in the range of 0.1 mg/day to 20 mg/day.

In embodiments, the pharmaceutical composition is sterile. In embodiments, the pharmaceutical composition is sterilized by conventional well-known techniques including, but not limited to, sterile filtration.

In embodiments, the pharmaceutical composition is used for administration to a subject.

In embodiments, the pharmaceutical composition is lyophilized prior to storage or stored as solution at ambient temperature or below, including, but not limited to, frozen storage.

In embodiments, the pharmaceutical composition is reconstituted and/or diluted in an infusion and stabilizer solution prior to administration to a subject. The solutions used for reconstitution or infusion/stabilization may contain any of the components mentioned for the pharmaceutical composition or similar components.

Another aspect of the invention is a kit comprising
a) a nucleic acid, vector and/or cell comprising a nucleotide sequence encoding the switchable CAR according to the invention, and
b) a targeting module comprising at least one target cell-binding domain capable of binding a target antigen and a tag or tag-binding domain or at least one nucleic acid, vector or cell encoding a targeting module,
wherein the tag-binding domain of the targeting module binds to the tag of the switchable chimeric antigen receptor or the tag of the targeting module binds to the tag-binding domain of the switchable chimeric antigen receptor.

In embodiments, the targeting module is isolated. As used herein, the term "isolated" means altered or removed from the natural state. In embodiments, the targeting module is expressed as a recombinant protein. In further embodiments, the targeting module is chemically synthesized.

In embodiments, the targeting module is in monomeric, dimeric or polymeric form, preferably in monomeric form.

As used herein, the term "target cell-binding domain" refers to a peptide, protein, or low molecular weight organic ligand, which specifically binds a protein or protein complex (antigen) on the surface of a target cell, in particular a cancer cell, T cell, infected cell, pathogens or parasites.

In embodiments, the at least one target cell-binding domain of the targeting module is an antibody, antigen-binding fragment, a protein, a peptide or a low molecular weight organic ligand that binds to a surface antigen selected from the group comprising CD2, CD3, CD4, CD5, CD7, CD8, CD10, CD15, CD19, CD20, CD22, CD23, CD25, CD30, CD33, CD38, CD44, CD44v6, CD52, CD66a, CD66b, CD66c, CD66d, CD66e, CD66f, CD79a, CD79b, CD90, CD99, CD123, CD133, CD135, CD150, CD181, CD182, CD184, CD223, CD229, CD269, CD273, CD274, CD276, CD279, CD319, CD366, CD371, a cytokine receptor, CXCR4, c-Met, mesothelin, a member of the epidermal growth factor receptor family or a mutant thereof, a member of the tumor necrosis factor receptor superfamily, a claudin, an ephrin, an ephrin receptor, a fucosyl transferase, a prostate specific antigen, an embryonic antigen, a member of the vascular endothelia growth factor family, epithelial cell adhesion molecule, alpha-fetoprotein, a member of the intercellular adhesion molecule family, a C-type lectin, an integrin, a member of the mucin protein family, a follicle-stimulating hormone receptor, a high molecular weight-melanoma associated antigen, a folate binding protein, a folate receptor, a somatostatin receptor, a ligand of the NKG2D receptor, a member of the epithelia glycoprotein family, a disialoganglioside, a glypican, a G protein-coupled receptor, a human papillomavirus protein, cancer/testis antigen, fibroblast activation protein, a member of the carbonic anhydrase family, a member of the carbohydrate antigen family, a Notch ligand, melanoma-associated chondroitin sulfate proteoglycan, glycoprotein A33, guanylate cyclase 2C and tumor-specific glycan.

The term "target cell-binding domain" also comprises soluble T cell receptors, which are composed of the alpha and beta or the gamma and delta chains of a T cell receptor (TCR), fragments or mutants thereof. Such TCR-derived binding moieties recognize and bind to peptides presented by human leukocyte antigen class (HLA) I and II protein complexes. Examples are, but are not limited to, TCRs specific for peptides derived from proteins like EGFR family, survivin, sry-like high motility group box (SOX) protein family, melanoma-associated antigens (e.g., autoimmunogenic cancer/testis antigen NY-ESO-1, members of the melanoma antigen family A MAGEA, the preferentially expressed antigen in melanoma PRAME), and leukemia-associated antigens (e.g. Wilms tumor gene 1 WT1).

In further embodiments, the target cell-binding domain is a soluble T cell receptor consisting of the alpha and beta or the gamma and delta chain of a TCR.

In embodiments, the at least one target cell-binding domain is an antibody, antibody fragment, a protein, a peptide or a low molecular weight organic ligand that binds to CD19, CD20, CD123, IL13Rα2, HER-2, PD-L1 and/or PD-L2.

In embodiments, the target cell-binding domain of the targeting module is an antibody or antigen-binding fragment.

In embodiments, the target cell-binding domain of the targeting module is an antigen-binding fragment. In embodiments, the target cell-binding domain is selected from single-chain variable fragments (scFv), single-chain antibodies, Fc fragment, F(ab')2 fragments, Fab fragments, and fragments produced by a Fab expression library or single-domain antibodies (nanobodies).

In embodiments, the at least one target cell-binding domain is an antibody fragment that binds to CD19, CD20, CD123, IL13Rα2, HER-2, PD-L1 and/or PD-L2.

In embodiments, the variable region(s) of the at least one target cell-binding domain comprises a humanized amino acid sequence.

In embodiments, the different domains of the targeting module are linked with each other by a linker. The linker comprises a short sequence of preferably 10 to 20 amino acid residues. In embodiments, the targeting module comprises a flexible peptide sequence that is selected such that the domains have a three-dimensional folding that allows them to exhibit the specificity for effector cell and target cell binding.

Preferred linkers are glycine-serine linkers with the structure (GₓS_{y}) with x and y selected from 1 to 10, preferably 1 to 5. Mostly preferred are 1 to 10 repeats of the sequence G₄S₁ (SEQ ID No. 20). Moreover, linkers are preferred that are constituted of a peptide sequence that can increase the protease resistance of the antibody derivatives. As used herein, the term "derivative" refers to a molecule (linker) having a high degree of structural identity to the molecule, preferably the same scaffold, wherein at least one atom, group of atoms, functional group or substructure is replaced with another atom, group of atoms functional group or substructure, e.g., a hydroxy group. Advantageously, the derivative is capable of performing one or more activities of the named molecule.

In embodiments, the linker is SEQ ID No. 21 or SEQ ID No. 22.

In embodiments, the targeting module comprises a further domain selected from the group comprising co-stimulatory ligands, radionuclides, cell death-inducing chemical compounds and half-life increasing domains, preferably IgG1 Fc, IgG2 Fc, IgG3 Fc, IgG4 Fc, HSA, FcRn-binding peptides or mutants thereof. As used herein, the term "mutants" refers to proteins having at least 90 % sequence identity to the half-life increasing domain, preferably at least 95 % sequence identity. Advantageously, the mutant is capable of having one or more activities of the named peptides or proteins; in particular, the mutant increases the half-life like the half-life increasing domain.

In preferred embodiments, the targeting module comprises a further domain selected from the group comprising co-stimulatory ligands, radionuclides, cell death-inducing chemical compounds and half-life increasing domains, preferably IgG1 Fc, IgG2 Fc, IgG3 Fc, IgG4 Fc, HSA or FcRn-binding peptides.

In embodiments, the tag-binding domain of the targeting module comprises at least one half-life increasing domain. In embodiments, the tag-binding domain comprises at least one half-life increasing domain selected from an amino acid sequence according to SEQ ID No. 143 or SEQ ID No. 144.

In embodiments, the tag-binding domain of the targeting module comprises at least one leader peptide. In embodiments, the tag-binding domain comprises at least one leader peptide selected from an amino acid sequence according to SEQ ID No. 145 or SEQ ID No. 146.

In embodiments, the length of the targeting module is in the range of 20 to 1600 amino acids, preferably 600 to 1600 amino acids.

In embodiments, the targeting module comprises two or three chains, wherein the length of the chains is independently from each other in the range of 200 to 550 amino acids In embodiments, the at least one target cell-binding domain is an antibody, antibody fragment, a protein, a peptide or a low molecular weight organic ligand that binds to CD19 and/or CD20.

In embodiments, the targeting module comprises
i) at least one CD19-binding domain comprising one of the sequences selected from SEQ ID No. 180 to SEQ ID No. 182 or a sequence identity of at least 95 % with one of the sequences selected from SEQ ID No. 180 to SEQ ID No. 182,
ii) at least one CD20-binding domain comprising SEQ ID No. 183 or one of the sequences selected from SEQ ID No. 184 to SEQ ID No. 186 or with a sequence identity of at least 95 % with one of the sequences selected from SEQ ID No. 184 to SEQ ID No. 186, and
iii) a tag-binding domain or a tag.

Advantageously, the trispecific targeting module targets both CD19 and CD20 simultaneously.

In embodiments, the targeting module comprises at least three chains comprising sequences from the group comprising SEQ ID No. 147 to SEQ ID No. 179.

In embodiments, the targeting module comprises at least three chains comprising
- one amino acid sequence selected from the group comprising SEQ ID No. 142, SEQ ID No. 149, SEQ ID No. 150, SEQ ID No. 175, SEQ ID No. 176, SEQ ID No. 177, SEQ ID No. 178, and SEQ ID No. 179, and
- one amino acid sequence selected from the group comprising SEQ ID No. 147 and SEQ ID No. 148, and
- one amino acid sequence selected from the group comprising SEQ ID No. 151, SEQ ID No. 152, SEQ ID No. 153, SEQ ID No. 154, SEQ ID No. 155, SEQ ID No. 156, SEQ ID No. 157, SEQ ID No. 158, SEQ ID No. 159, SEQ ID No. 160, SEQ ID No. 161, SEQ ID No. 162, SEQ ID No. 163, SEQ ID No. 164, SEQ ID No. 165, SEQ ID No. 166, SEQ ID No. 167, SEQ ID No. 168, SEQ ID No. 169, SEQ ID No. 170, SEQ ID No. 171, SEQ ID No. 172, SEQ ID No. 173, and SEQ ID No. 174.

In embodiments, the targeting module comprises
- SEQ ID No. 149, SEQ ID No.147 and SEQ ID No. 151, or
- SEQ ID No. 150, SEQ ID No. 148 and SEQ ID No. 152, or
- SEQ ID No. 149, SEQ ID No. 147 and SEQ ID No. 153, or
- SEQ ID No. 150, SEQ ID No. 148 and SEQ ID No. 154, or
- SEQ ID No. 149, SEQ ID No. 147 and SEQ ID No. 155, or
- SEQ ID No. 150, SEQ ID No. 148 and SEQ ID No. 156, or
- SEQ ID No. 149, SEQ ID No. 147 and SEQ ID No. 157, or
- SEQ ID No. 150, SEQ ID No. 148 and SEQ ID No. 158, or
- SEQ ID No. 149, SEQ ID No. 147 and SEQ ID No. 159, or
- SEQ ID No. 150, SEQ ID No. 148 and SEQ ID No. 160, or
- SEQ ID No. 149, SEQ ID No. 147 and SEQ ID No. 161, or
- SEQ ID No. 150, SEQ ID No. 148 and SEQ ID No. 162, or
- SEQ ID No. 142, SEQ ID No. 147 and SEQ ID No. 163, or
- SEQ ID No. 179, SEQ ID No. 148 and SEQ ID No. 164, or
- SEQ ID No. 142, SEQ ID No. 147 and SEQ ID No. 165, or
- SEQ ID No. 179, SEQ ID No. 148 and SEQ ID No. 166, or
- SEQ ID No. 142, SEQ ID No. 147 and SEQ ID No. 167, or
- SEQ ID No. 179, SEQ ID No. 148 and SEQ ID No. 168, or
- SEQ ID No. 142, SEQ ID No. 147 and SEQ ID No. 169, or
- SEQ ID No. 179, SEQ ID No. 148 and SEQ ID No. 170, or
- SEQ ID No. 142, SEQ ID No. 147 and SEQ ID No. 171, or
- SEQ ID No. 179, SEQ ID No. 148 and SEQ ID No. 172, or
- SEQ ID No. 142, SEQ ID No. 147 and SEQ ID No. 173, or
- SEQ ID No. 179, SEQ ID No. 148 and SEQ ID No. 174, or
- SEQ ID No. 175, SEQ ID No. 147 and SEQ ID No. 157, or
- SEQ ID No. 176, SEQ ID No. 148 and SEQ ID No. 158, or
- SEQ ID No. 177, SEQ ID No. 147 and SEQ ID No. 157, or
- SEQ ID No. 178, SEQ ID No. 148 and SEQ ID No. 158, or
- SEQ ID No. 175, SEQ ID No. 147 and SEQ ID No. 159, or
- SEQ ID No. 176, SEQ ID No. 148 and SEQ ID No. 160, or
- SEQ ID No. 177, SEQ ID No. 147 and SEQ ID No. 159, or
- SEQ ID No. 178, SEQ ID No. 148 and SEQ ID No. 160, or
- SEQ ID No. 175, SEQ ID No. 147 and SEQ ID No. 161, or
- SEQ ID No. 176, SEQ ID No. 148 and SEQ ID No. 162, or
- SEQ ID No. 177, SEQ ID No. 147 and SEQ ID No. 161, or
- SEQ ID No. 178, SEQ ID No. 148 and SEQ ID No. 162, or
- SEQ ID No. 175, SEQ ID No. 147 and SEQ ID No. 151, or
- SEQ ID No. 176, SEQ ID No. 148 and SEQ ID No. 152, or
- SEQ ID No. 177, SEQ ID No. 147 and SEQ ID No. 151, or
- SEQ ID No. 178, SEQ ID No. 148 and SEQ ID No. 152, or
- SEQ ID No. 175, SEQ ID No. 147 and SEQ ID No. 153, or
- SEQ ID No. 176, SEQ ID No. 148 and SEQ ID No. 154, or
- SEQ ID No. 177, SEQ ID No. 147 and SEQ ID No. 153, or
- SEQ ID No. 178, SEQ ID No. 148 and SEQ ID No. 154, or
- SEQ ID No. 175, SEQ ID No. 147 and SEQ ID No. 155, or
- SEQ ID No. 176, SEQ ID No. 148 and SEQ ID No. 156, or
- SEQ ID No. 177, SEQ ID No. 147 and SEQ ID No. 155, or
- SEQ ID No. 178, SEQ ID No. 148 and SEQ ID No. 156.

In embodiments, the at least one target cell-binding domain is an antibody, antibody fragment, a protein, a peptide or a low molecular weight organic ligand that binds to CD123.

In embodiments, the targeting module according to the invention comprises
i) at least one CD123-binding domain comprising a sequence with at least 95 % identity, preferably 99 % identity, with the sequence according to SEQ ID No. 187 and SEQ ID No. 188, and
ii) a tag-binding domain or a tag.

In embodiments, the targeting module according to the invention comprises
i) at least one CD123-binding domain comprising a sequence with at least 95 % identity, preferably 99 % identity, with the sequence according to SEQ ID No. 187 and SEQ ID No. 188, and
ii) a tag-binding domain binding a human La epitope E5B9 comprising a V_{L}-linker-V_{H} structure, wherein the V_{L} region of the tag-binding domain comprises a sequence with at least 95 % identity, preferably 99 % identity, with the sequence according to SEQ ID No. 14 and/or the V_{H} region of the tag-binding domain comprises a sequence with at least 95 % identity, preferably 99 % identity, with the sequence according to SEQ ID No. 15.

In embodiments, the CD123-binding domain comprises the sequences SEQ ID No. 187 and SEQ ID No. 188.

In preferred embodiments, the CD123-binding domain comprises a sequence according to SEQ ID No. 189.

In embodiments, the targeting module comprises one of the sequences according to SEQ ID No. 190 to SEQ ID No. 197.

In alternative embodiments, the kit comprises a nucleic acid, vector or cell encoding the targeting module. The nucleic acid, vector and/or cell are isolated.

According to the invention, the nucleic acid, vector and/or cell comprising a nucleotide sequence encoding the switchable CAR according to the invention are isolated.

In embodiments, the nucleic acid is a cDNA. As used herein, the term "cDNA" (complementary DNA) refers to double-stranded DNA synthesized from a single-stranded RNA, e.g. mRNA, in a reaction catalyzed by the enzyme reverse transcriptase. In embodiments, cDNA is of synthetic origin. In further embodiments, cDNA is derived from mRNA, therefore containing only exons but no introns, as opposed to genomic DNA.

The vector is preferably a plasmid, an artificial chromosome, linearized DNA or RNA, a virus particle or another vector that contains an expression cassette that is incorporated stably into the genome of a host cell or host organism.

In embodiments, the cell is selected from immune cells, preferably with cytolytic, phagocytic or immunosuppressive activity, such as T cells, Natural Killer (NK) cells and macrophages. In preferred embodiments, the cell is selected from T cells, including alpha/beta and gamma/delta T cells or subpopulations of T cells like stem-cell memory T cells or central memory T cells, cytotoxic T cells or NK cells.

In embodiments, the cell comprising a nucleotide sequence encoding a reversible chimeric antigen receptor and/or the targeting module are in the form of a pharmaceutical composition.

In embodiments, the kit further comprises at least one further targeting module or at least one further nucleic acid, vector or cell encoding a further targeting module,
wherein the at least one further targeting module comprises at least one target cell-binding domain and a tag-binding domain or a tag,
wherein the at least one target cell-binding domain of the further targeting module is an antibody, antigen-binding fragment, a protein, a peptide or a low molecular weight organic ligand that binds to a surface antigen selected from the group comprising CD2, CD3, CD4, CD5, CD7, CD8, CD10, CD15, CD19, CD20, CD22, CD23, CD25, CD30, CD33, CD38, CD44, CD44v6, CD52, CD66a, CD66b, CD66c, CD66d, CD66e, CD66f, CD79a, CD79b, CD90, CD99, CD123, CD133, CD135, CD150, CD181, CD182, CD184, CD223, CD229, CD269, CD273, CD274, CD276, CD279, CD319, CD366, CD371, cytokine receptors, CXCR4, c-Met, mesothelin, a member of the epidermal growth factor receptor family or a mutant thereof, a member of the tumor necrosis factor receptor superfamily, a claudin, an ephrin, an ephrin receptor, a fucosyl transferase, a prostate specific antigen, an embryonic antigen, a member of the vascular endothelia growth factor family, epithelial cell adhesion molecule, alpha-fetoprotein, a member of the intercellular adhesion molecule family, a C-type lectin, an integrin, a member of the mucin protein family, a follicle-stimulating hormone receptor, a high molecular weight-melanoma associated antigen, a folate binding protein, a folate receptor, a somatostatin receptor, a ligand of the NKG2D receptor, a member of the epithelia glycoprotein family, a disialoganglioside, a glypican, a G protein-coupled receptor, a human papillomavirus protein, cancer/testis antigen, fibroblast activation protein, a member of the carbonic anhydrase family, a member of the carbohydrate antigen family, a Notch ligand, melanoma-associated chondroitin sulfate proteoglycan, glycoprotein A33, guanylate cyclase 2C and tumor-specific glycan,
wherein the targeting module and the at least one further targeting module comprise different target cell-binding domains, and identical tag-binding domains or tags.

In embodiments, the kit comprises one to three targeting modules.

In embodiments, the kit comprises one to three targeting modules targeting CD33, CD123 and CD371, or CD19, CD20 and CD22, or CD19, CD38 and CD269.

In embodiments, the kit comprises one to five targeting modules targeting CD38, CD269, GPRC5D, CD79a and CD79b.

In embodiments, the switchable CAR, the cell, the pharmaceutical composition or the kit is used as a medicament.

In embodiments, the switchable CAR according to the invention, the nucleic acid, vector or cell according to the invention, the pharmaceutical composition according to the invention or the kit according to the invention is used for preparing a medication for therapeutic and/or diagnostic use in case of cancer, an infection or an autoimmune disease.

The term "autoimmune disorder" refers to an abnormal immune response of the body against substances and tissues normally present in the body (autoimmunity).

In embodiments, the switchable CAR, the cell, the pharmaceutical composition or the kit is used in the treatment of cancer, infectious disease or autoimmune disease.

In embodiments, the switchable CAR according to the invention, the cell, the pharmaceutical composition or the kit is used in the treatment of cancer. In embodiments, the switchable CAR according to the invention is used in the treatment of tumors of the hematopoietic and lymphoid tissues. In embodiments, the switchable CAR according to the invention is used in the treatment of blood cancer. In embodiments, the switchable CAR according to the invention is used in the treatment of b cell lymphomas.

In embodiments, the cell according to the invention is administered in combination with a targeting module, wherein the tag of the switchable CAR binds to the tag-binding domain of the targeting module or the tag-binding domain of the switchable CAR binds to the tag of the targeting module.

As used herein, the term "administered in combination" refers to a treatment, wherein the targeting module is administered prior to, simultaneously with and/or after the administration of the cell comprising a nucleotide sequence encoding the switchable CAR.

In embodiments, the switchable CAR according to the invention and a targeting module are used in a method for treating cancer, infectious disease or autoimmune disease in a subject in need thereof.

For therapeutic applications, a sterile pharmaceutical composition according to the invention or a sterile kit according to the invention, comprising a pharmacologically effective quantity of the cell comprising a nucleotide sequence encoding a switchable CAR according to the invention and a targeting module, is administered to a subject in order to treat the aforementioned illnesses.

In embodiments, the method for stimulating a CAR-mediated immune response in a mammal; preferably, the method for treatment of cancer, infectious or autoimmune disease, comprises the following steps:
a) administering to a mammal an effective amount of a targeting module and
b) administering to the mammal an effective amount of a cell comprising a nucleotide sequence encoding a switchable CAR according to the invention,
   wherein the tag of the switchable CAR binds to the tag-binding domain of the targeting module or the tag-binding domain of the switchable CAR binds to the tag of the targeting module,
wherein the targeting module is administered to a mammal prior to, concurrent with or after the administration of the cell.

In embodiments, the targeting module is administered one hour to 2 days, preferably 4 to 24 hours, prior to the administration of the cell comprising a nucleotide sequence encoding the switchable CAR. Advantageously, the administration of the targeting module prior to the administration of the cell comprising the nucleotide sequence encoding the switchable CAR stimulates the switchable CAR and increases the expansion of the switchable CAR carrying effector cells and their accumulation at the target site.

In embodiments, the targeting module is administered simultaneously with the cell comprisingthe nucleotide sequence encoding a switchable CAR.

In embodiments, the targeting module is administered until, preferably in the range of 3days to 30 days, after the administration of the cell comprising the nucleotide sequence encoding the switchable CAR. In embodiments, additional such doses of the targeting module may be administered following resting periods to reactivate the switchable CAR-carrying effector cells.

In embodiments, a dosage of the targeting module is continuously administered to a subject having cancer, in particular hematological cancer, with a dosage quantity in the range of 0.01 mg/day to 100 mg/day from day 0 of the treatment (beginning of the treatment) for a period between 10 days and 180 days, preferably, between 10 days and 25 days, and at least one dosage of a clinically effective amount of the T cell comprising a nucleotide sequence encoding a switchable CAR is administered to the subject with a dosage quantity in the range of 1·10⁸ to 1·10⁹ cells once within day 0 to day 5 of the treatment.

In embodiments, a first dosage of the targeting module is continuously administered to a subject having cancer, in particular hematological cancer, with a dosage quantity in the range of 0.01 mg/day to 100 mg/day from day 0 of the treatment (beginning of the treatment) for a period between 10 days and 25 days, and at least one dosage of a clinically effective amount of the T cell comprising a nucleotide sequence encoding a switchable CAR is administered to the subject with a dosage quantity in the range of 1·10⁸ to 1·10⁹ cells once within day 0 to day 5 of the treatment, and wherein 7 days to 21 days after the administering of the first dosage of the targeting module at least one further dosage of the targeting module is continuously administered to the subject with a dosage quantity in the range of 0.01 mg/day to 100 mg/day for a period between 4 days and 25 days.

In embodiments, targeting module is administered to a subject having cancer, in particular hematological cancer, from day 0 of the treatment (beginning of the treatment) for a period between 7 days and 180 days, preferably between 7 days and 28 days.

In embodiments, targeting module is administered by 1 to 7 injections or infusions per week or each once a week to a subject having cancer, in particular hematological cancer.

In embodiments, targeting module is administered with a dosage quantity in the range of 0.01 mg/day to 100 mg/day.

In embodiments, at least one dosage of a clinically effective amount of the T cell comprising a nucleotide sequence encoding a switchable CAR is administered to the subject with a dosage quantity in the range of 1·10⁸ to 1·10⁹ cells once within day 0 to day 5 of the treatment.

In embodiments, targeting module is administered by 1 to 7 bolus injections or short infusions per week or each once a week to a subject having cancer.

As used herein, the term "bolus" refers to the administration of a discrete amount of targeting module within a specific time, in particular 1 to 30 minutes.

The invention is not limited to the specifically described combinations of features but may also be defined by any other combination of specific features of all the individual features disclosed as a whole, provided that the individual features are not mutually exclusive, or a specific combination of individual features is not explicitly excluded.

In the following, the invention will be explained in more detail by means of an embodiment example. The embodiment example is intended to describe the invention without limiting it. Implementations of the invention will be described, by way of example only, with reference to accompanying drawings in which:

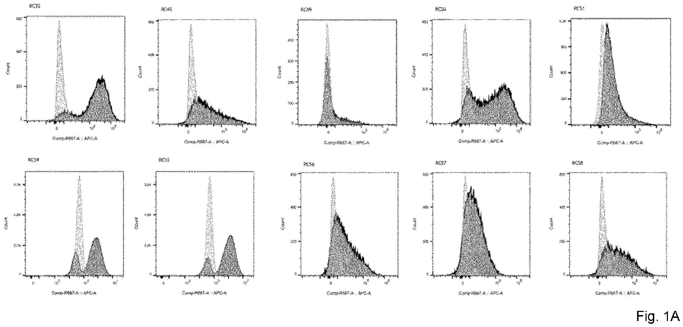

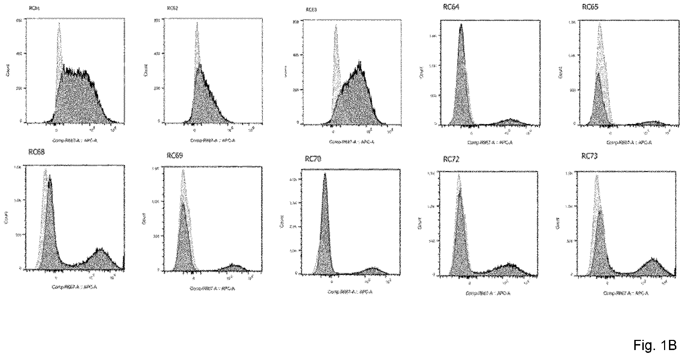

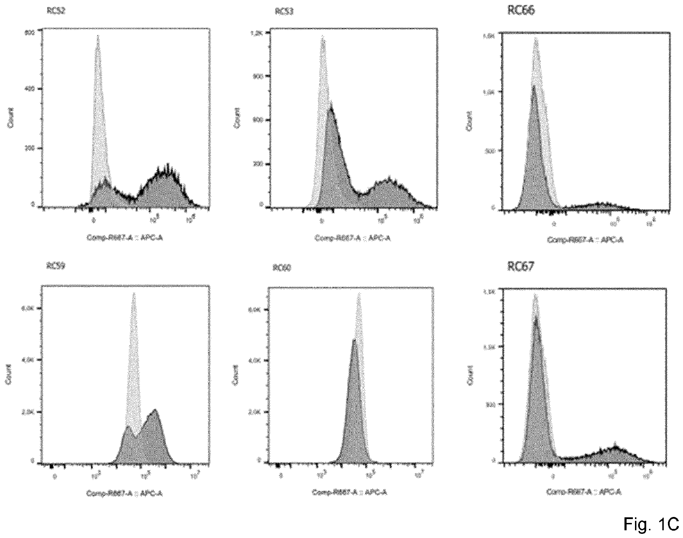

**Fig. 1A****,** **1B** **and** **1C** show switchable CAR (RevCAR) surface detection on transduced primary human T cells by flow cytometry. Transduced primary human T cells were characterized after manufacturing by staining with mouse anti-human La/SSB mab (clone 5B9) conjugated to AF647. For each CAR construct one representative product is depicted. Isolated T cells were transduced with lentivirus encoding for the respective switchable CAR construct and TRAC was knocked out using CRISPR/Cas9. Isotype control is shown in light grey. Fluorescence for mab 5B9 AF647 is shown in dark grey.

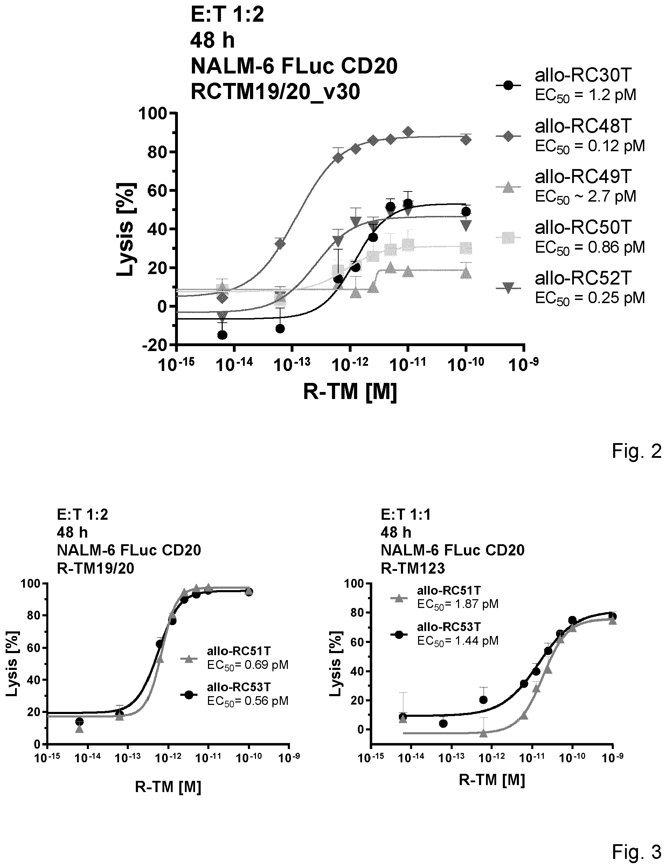

**Fig. 2** shows in vitro potency assay results for allogeneic switchable CAR (allo-RevCAR) T cells against Nalm6-CD20. Co-culture assay of allo-RevCAR T cell products with target cell line Nalm6-CD20 in presence of varying concentrations of anti-CD19/CD20 targeting module (R-TM19/20). Target cells were stained beforehand with the proliferation dye efluor 450. After 48 hrs, viable target cell count is determined by flow cytometry, dead cells are discriminated with propidium iodide and specific lysis is calculated (relative to control without targeting module). Mean of three replicates is plotted and fitted with a 4-parameter logistic regression. Calculated EC50 values represent the sensitivity of switchable CAR T products to the targeting module used.
**Fig. 3** shows in vitro potency assay results for switchable CAR (allo-RevCAR) T cells against Nalm6-CD20. Co-culture assay of allo-RevCAR T cell products with target cell line Nalm6-CD20 in presence of varying concentrations of anti-CD19/CD20 targeting module (R-TM19/20) or anti-CD123 targeting module (R-TM123), respectively. Target cells were stained beforehand with the proliferation dye efluor 450. After 48 hrs, viable target cell count is determined by flow cytometry, dead cells are discriminated with propidium iodide and specific lysis is calculated (relative to control without targeting module). Mean of three replicates is plotted and fitted with a 4-parameter logistic regression. Calculated EC50 values represent the sensitivity of RevCAR Tcell products to the targeting module used.

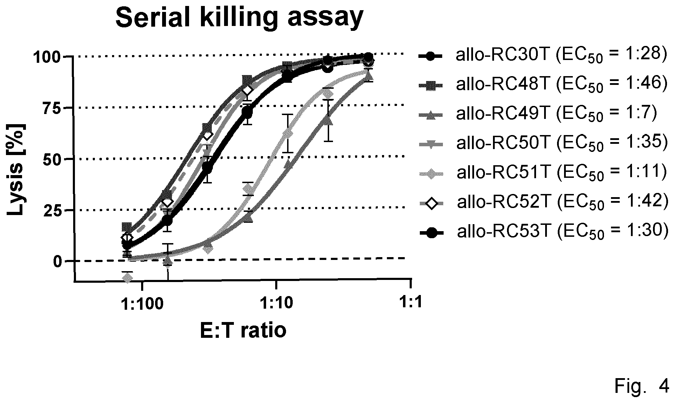

**Fig. 4** shows results of a serial killing assay results of allogeneic switchable CAR (allo-RevCAR) T cells against Nalm6-CD20 cells. Co-culture assay of allo-RevCAR T cell products with target cell line Nalm6-CD20 in presence of 1 nM anti-CD19/CD20 targeting module (R-TM 19/20). Target cells were stained beforehand with the proliferation dye efluor 450. After 48 hrs, viable target cell count is determined by flow cytometry, dead cells are discriminated with propidium iodide and specific lysis is calculated (relative to control without targeting module). Mean of three replicates is plotted and fitted with a 4-parameter logistic regression. Calculated EC50 values represent the capacity of RevCAR T products to kill multiple target cells.

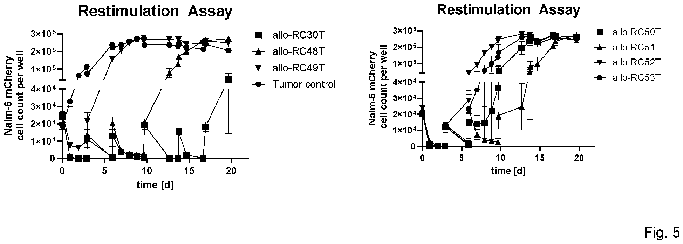

**Fig. 5** shows results of a long-term restimulation assay results of allogeneic switchable CAR (allo-RevCAR) T cells against Nalm6 cells. Co-culture assay of allo-RevCAR T cell products with target cell line Nalm6 (mcherry positive) in presence of 0.2 nM anti-CD19/CD20 targeting module (R-TM 19/20). Every 3 to 4 days 2·10⁴ fresh target cells are added. Mcherry positive target cell counts are determined by fluorescence microscopy (Incucyte S3) twice a day over three weeks. Mean and standard deviation of four replicates is plotted.

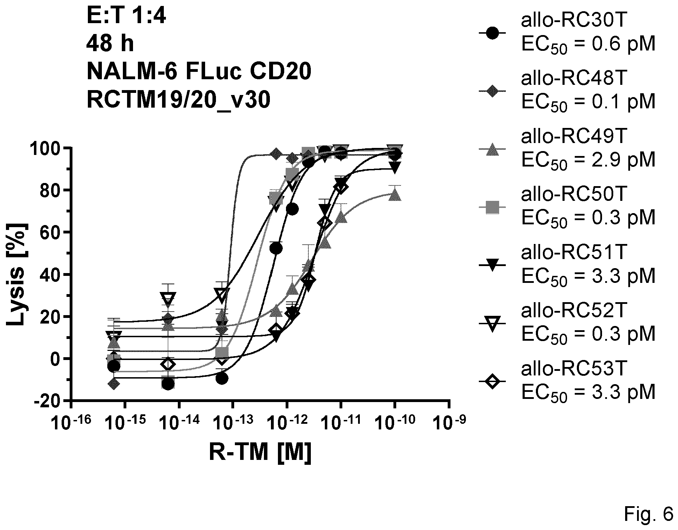

**Fig. 6** shows in vitro potency assay results for allogeneic switchable CAR (allo-RevCAR) T cells against Nalm6-CD20. Co-culture assay of allo-RevCAR T cell products with target cell line Nalm6-CD20 in presence of varying concentrations of anti-CD19/CD20 targeting module (R-TM19/20). Target cells were stained beforehand with the proliferation dye efluor 450. After 48 hrs, viable target cell count is determined by flow cytometry, dead cells are discriminated with propidium iodide and specific lysis is calculated (relative to control without R-TM). Mean of three replicates is plotted and fitted with a 4-parameter logistic regression. Calculated EC50 values represent the sensitivity of RevCAR T cell products to the targeting module used.

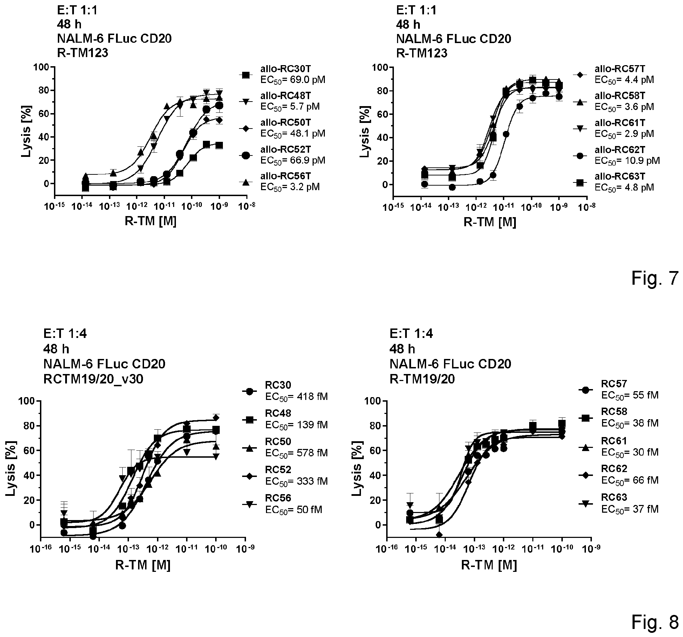

**Fig. 7** shows in vitro potency assay results for allogeneic switchable CAR (allo-RevCAR) T cells against Nalm6-CD20. Co-culture assay of allo-RevCAR T cell products with target cell line Nalm6-CD20 in presence of varying concentrations of anti-CD123 targeting module (R-TM123). Target cells were stained beforehand with the proliferation dye efluor 450. After 48 hrs, viable target cell count is determined by flow cytometry, dead cells are discriminated with propidium iodide and specific lysis is calculated (relative to control without targeting module). Mean of three replicates is plotted and fitted with a 4-parameter logistic regression. Calculated EC50 values represent the sensitivity of RevCAR T cell products to the targeting module used.
**Fig. 8** shows in vitro potency assay results for allogeneic switchable CAR (allo-RevCAR) T cells against Nalm6-CD20. Co-culture assay of allo-RevCAR T cell products with target cell line Nalm6-CD20 in presence of varying concentrations of anti-CD19/CD20 targeting module (R-TM19/20). Target cells were stained beforehand with the proliferation dye efluor 450. After 48hrs, viable target cell count is determined by flow cytometry, dead cells are discriminated with propidium iodide and specific lysis is calculated (relative to control without targeting module). Mean of three replicates is plotted and fitted with a 4-parameter logistic regression. Calculated EC50 values represent the sensitivity of RevCAR T cell products to the targeting module used.

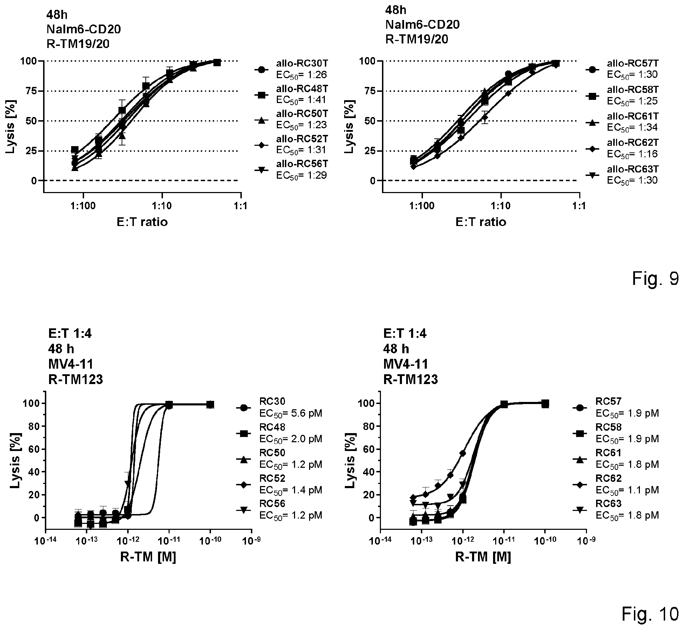

**Fig. 9** shows serial killing assay results of allogeneic switchable CAR (allo-RevCAR) T cells against Nalm6-CD20 cells. Co-culture assay of allo-RevCAR T cell products with target cell line Nalm6-CD20 in presence of 1 nM anti-CD19/CD20 targeting module (R-TM19/20). Target cells were stained beforehand with the proliferation dye efluor 450. After 48 hrs, viable target cell count is determined by flow cytometry, dead cells are discriminated with propidium iodide and specific lysis is calculated (relative to control without targeting module). Mean of three replicates is plotted and fitted with a 4-parameter logistic regression. Calculated EC50 values represent the capacity of RevCAR T cells products to kill multiple target cells.
**Fig. 10** shows in vitro potency assay results for allogeneic switchable CAR (allo-RevCAR) T against MV4-11. Co-culture assay of allo-RevCAR T cell products with target cell line MV4-11 in presence of varying concentrations ofanti-CD123 targeting module (R-TM123). Target cells were stained beforehand with the proliferation dye efluor 450. After 48 hrs, viable target cell count is determined by flow cytometry, dead cells are discriminated with propidium iodide and specific lysis is calculated (relative to control without targeting module). Mean of three replicates is plotted and fitted with a 4-parameter logistic regression. Calculated EC50 values represent the sensitivity of RevCAR T cell products to the targeting module used.

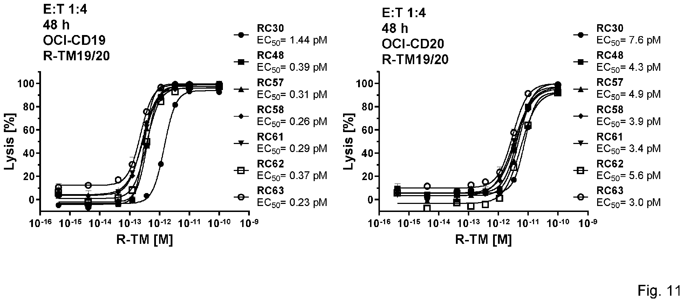

**Fig. 11** shows in vitro potency assay results for allogeneic switchable CAR (allo-RevCAR) T cells against Oci-AML-CD19 and Oci-AML-CD20. Co-culture assay of allo-RevCAR T cell products with target cell line Oci-AML-CD19 and Oci-AML-CD20 in presence of varying concentrations of anti-CD19/CD20 targeting module (R-TM19/20). Target cells were stained beforehand with the proliferation dye efluor 450. After 48 hrs, viable target cell count is determined by flow cytometry, dead cells are discriminated with propidium iodide and specific lysis is calculated (relative to control without targeting module). Mean of three replicates is plotted and fitted with a 4-parameter logistic regression. Calculated EC50 values represent the sensitivity of RevCAR Tcell products to the targeting module used.

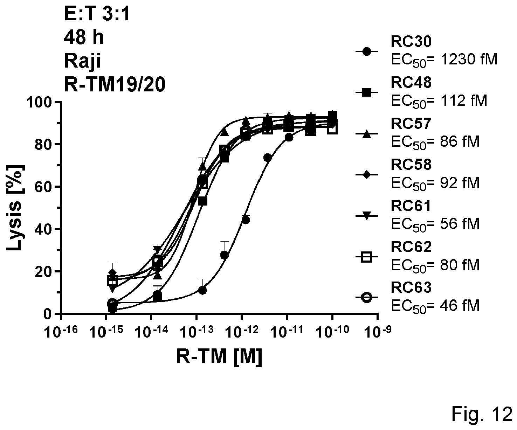

**Fig. 12** shows in vitro potency assay results for allogeneic switchable CAR (allo-RevCAR) T cells against Raji lymphoma cells. Co-culture assay of allo-RevCAR T cell products with target cell line Raji in presence of varying concentrations of anti-CD19/CD20 targeting module (R-TM19/20). Target cells were stained beforehand with the proliferation dye efluor 450. After 48hrs, viable target cell count is determined by flow cytometry, dead cells are discriminated with propidium iodide and specific lysis is calculated (relative to control without targeting module). Mean of three replicates is plotted and fitted with a 4-parameter logistic regression. Calculated EC50 values represent the sensitivity of RevCAR T cell products to the targeting module used.

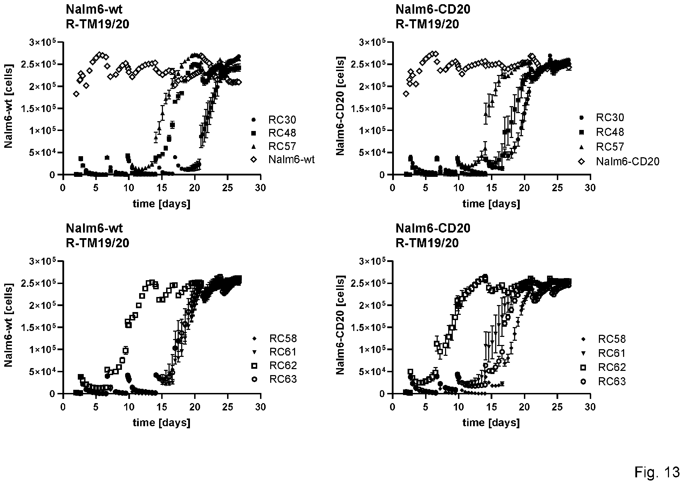

**Fig. 13** shows in Long-term restimulation assay results of allogeneic switchable CAR (allo-RevCAR) T cells against Nalm6 and Nalm6-CD20 cells. Co-culture assay of allo-RevCAR T cell products with target cell line Nalm6 (mcherry positive) and Nalm6-CD20 (mcherry positive) in presence of 0.2 nM anti-CD19/CD20 targeting module (R-TM 19/20). Every 3 to 4 days 2·10⁴ fresh target cells are added. Mcherry positive target cell counts are determined by fluorescence microscopy (Incucyte S3) twice a day over three weeks. Mean and standard deviation of three replicates is plotted.

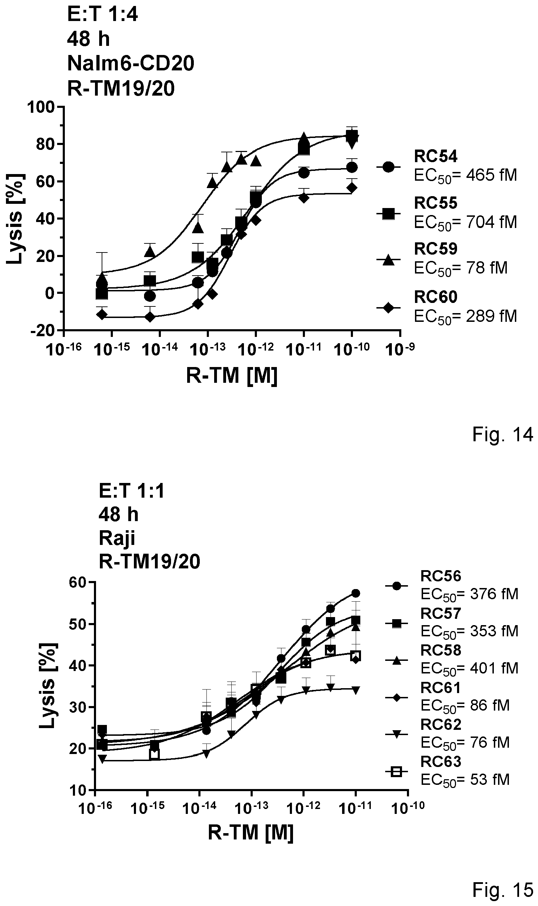

**Fig. 14** shows in vitro potency assay results for allogeneic switchable CAR (allo-RevCAR) T cells against Nalm6-CD20. Co-culture assay of allo-RevCAR T cell products with target cell line Nalm6-CD20 in presence of varying concentrations of anti-CD19/CD20 targeting module (R-TM19/20). Target cells were stained beforehand with the proliferation dye efluor 450. After 48hrs, viable target cell count is determined by flow cytometry, dead cells are discriminated with propidium iodide and specific lysis is calculated (relative to control without targeting module). Mean of three replicates is plotted and fitted with a 4-parameter logistic regression. Calculated EC50 values represent the sensitivity of RevCAR T cell products to the targeting module used.
**Fig. 15** shows in vitro potency assay results for allogeneic switchable CAR (allo-RevCAR) T cells against Raji lymphoma cells. Co-culture assay of allo-RevCAR T cell products with target cell line Raji in presence of varying concentrations of anti-CD19/CD20 targeting module (R-TM19/20). Target cells were stained beforehand with the proliferation dye efluor 450. After 48 hrs, viable target cell count is determined by flow cytometry, dead cells are discriminated with propidium iodide and specific lysis is calculated (relative to control without targeting module). Mean of three replicates is plotted and fitted with a 4-parameter logistic regression. Calculated EC50 values represent the sensitivity of RevCAR T cell products to the targeting module used.

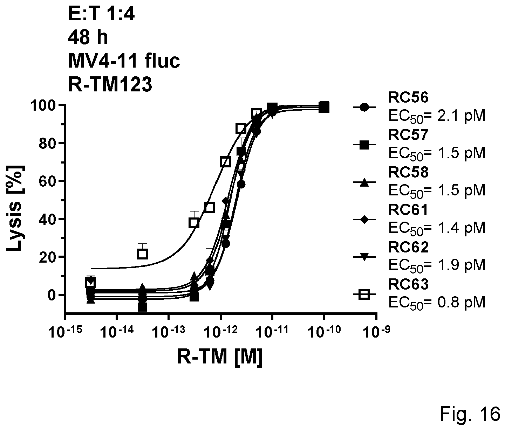

**Fig. 16** shows in vitro potency assay for allogeneic switchable CAR (allo-RevCAR) T cells against MV4-11. Co-culture assay of allo-RevCAR T cell products with target cell line MV4-11 in presence of varying concentrations of anti-CD123 targeting module (R-TM123). Target cells were stained beforehand with the proliferation dye efluor 450. After 48 hrs, viable target cell count is determined by flow cytometry, dead cells are discriminated with propidium iodide and specific lysis is calculated (relative to control without targeting module). Mean of three replicates is plotted and fitted with a 4-parameter logistic regression. Calculated EC50 values represent the sensitivity of RevCAR T cell products to the targeting module used.

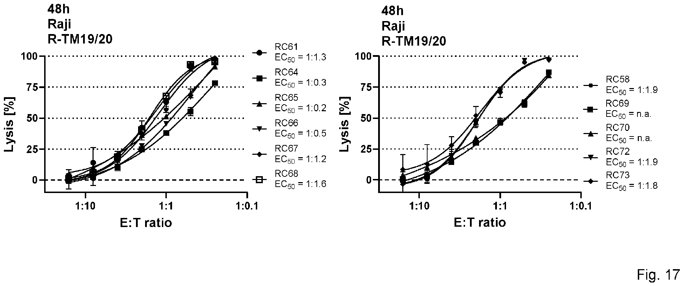

**Fig. 17** shows serial killing assay of allogeneic switchable CAR (allo-RevCAR) T cells against Raji lymphoma cells. Co-culture assay of allo-RevCAR T cell products with target cell line Raji in presence of 0.1 nM anti-CD19/CD20 targeting module (R-TM19/20). Target cells were stained beforehand with the proliferation dye efluor 450. After 48 hrs, viable target cell count is determined by flow cytometry, dead cells are discriminated with propidium iodide and specific lysis is calculated (relative to control without targeting module). Mean of three replicates is plotted and fitted with a 4-parameter logistic regression. Calculated EC50 values represent the capacity of RevCAR T cell products to kill multiple target cells.

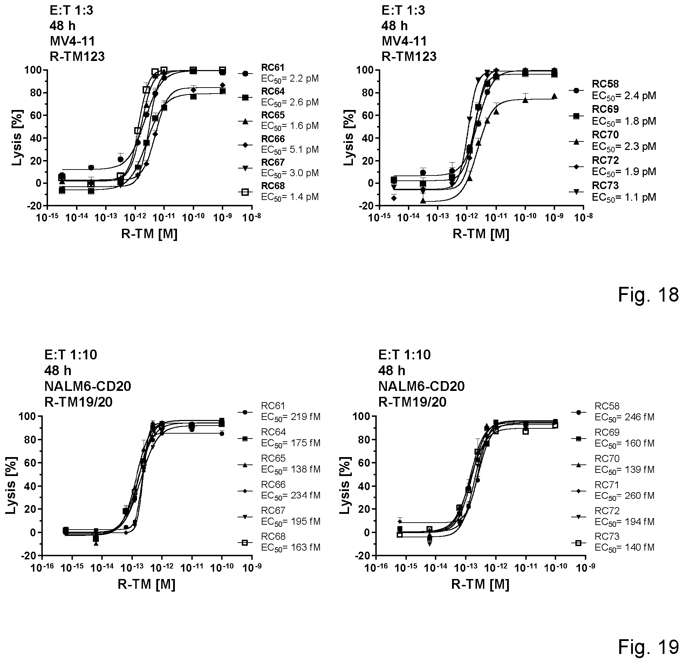

**Fig. 18** shows in vitro potency assay results for allogeneic switchable CAR (allo-RevCAR) T cells against MV4-11. Co-culture assay of allo-RevCAR T cell products with target cell line MV4-11 in presence of varying concentrations ofanti-CD123 targeting module (R-TM123). Target cells were stained beforehand with the proliferation dye efluor 450. After 48 hrs, viable target cell count is determined by flow cytometry, dead cells are discriminated with propidium iodide and specific lysis is calculated (relative to control without targeting module). Mean of three replicates is plotted and fitted with a 4-parameter logistic regression. Calculated EC50 values represent the sensitivity of RevCAR T cell products to the targeting module used.
**Fig. 19** shows in vitro potency assay results for allogeneic switchable CAR (allo-RevCAR) T cells against Nalm6-CD20. Co-culture assay of allo-RevCAR T cell products with target cell line Nalm6-CD20 in presence of varying concentrations of anti-CD19/CD20 targeting module (R-TM19/20). Target cells were stained beforehand with the proliferation dye efluor 450. After 48 hrs, viable target cell count is determined by flow cytometry, dead cells are discriminated with propidium iodide and specific lysis is calculated (relative to control without targeting module). Mean of three replicates is plotted and fitted with a 4-parameter logistic regression. Calculated EC50 values represent the sensitivity of RevCAR T cell products to the targeting module used.

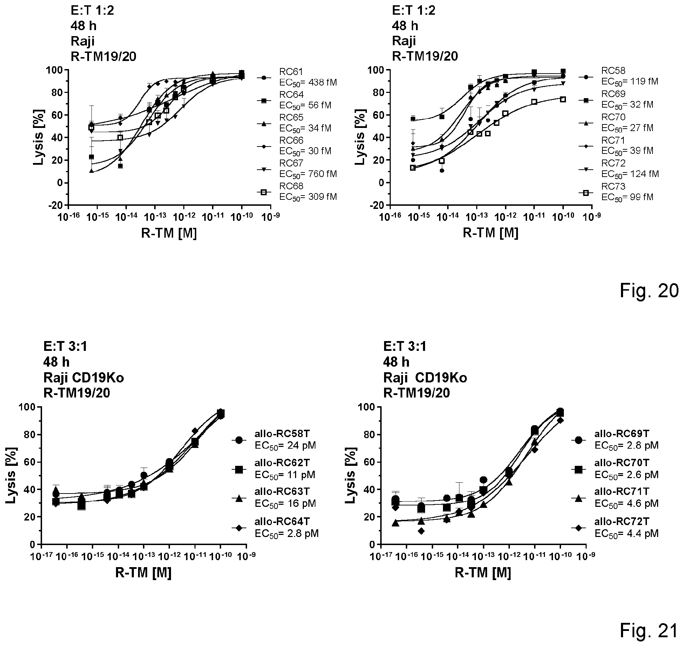

**Fig. 20** shows in vitro potency assay results for allogeneic switchable CAR (allo-RevCAR) T cells against Raji lymphoma cells. Co-culture assay of allo-RevCAR T cell products with target cell line Raji in presence of varying concentrations of anti-CD19/CD20 targeting module (R-TM19/20). Target cells were stained beforehand with the proliferation dye efluor 450. After 48 hrs, viable target cell count is determined by flow cytometry, dead cells are discriminated with propidium iodide and specific lysis is calculated (relative to control without targeting module). Mean of three replicates is plotted and fitted with a 4-parameter logistic regression. Calculated EC50 values represent the sensitivity of RevCAR T cell products to the targeting module used.
**Fig. 21** shows in vitro potency assay results for allogeneic switchable CAR (allo-RevCAR) T cells against Raji CD19Ko lymphoma cells. Co-culture assay of allo-RevCAR T cell products with target cell line Raji CD19Ko (negative for CD19) in presence of varying concentrations of anti-CD19/CD20 targeting module (R-TM19/20). Target cells were stained beforehand with the proliferation dye efluor 450. After 48 hrs, viable target cell count is determined by flow cytometry, dead cells are discriminated with propidium iodide and specific lysis is calculated (relative to control without targeting module). Mean of three replicates is plotted and fitted with a 4-parameter logistic regression. Calculated EC50 values represent the sensitivity of RevCAR Tcell products to the targeting module used.

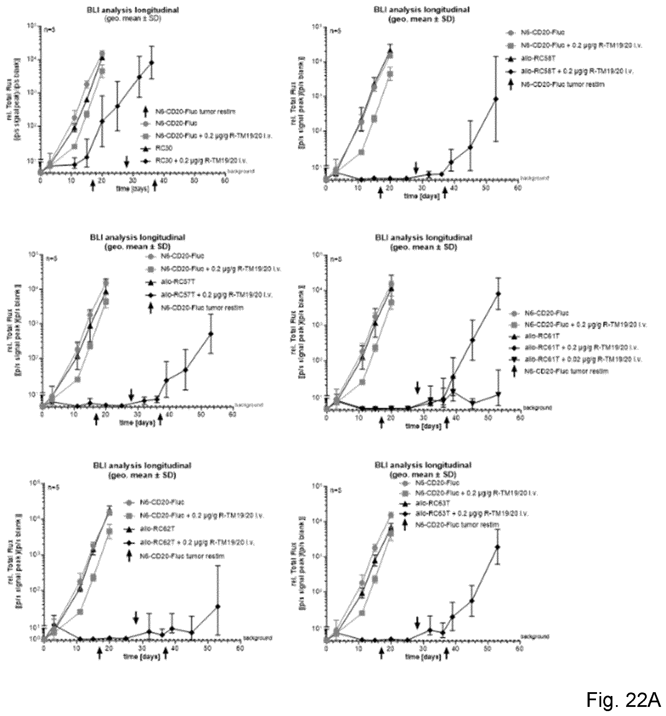

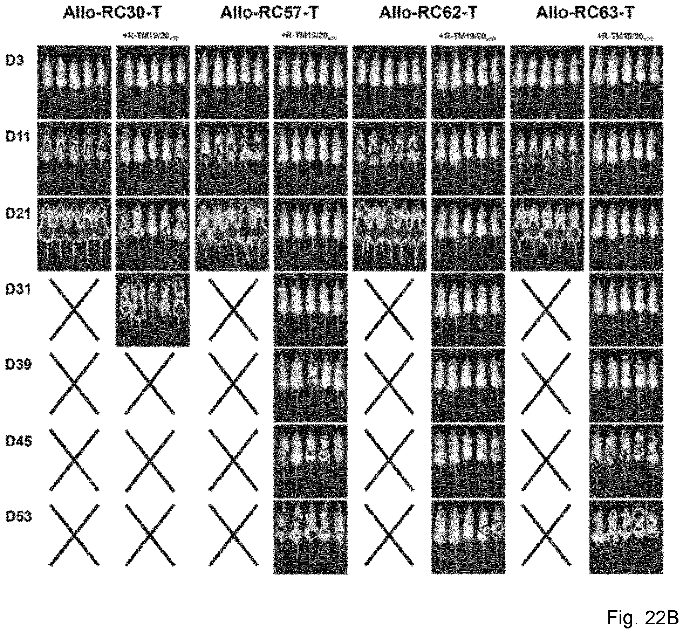

**Fig. 22A** **and** **22B** show in vivo efficacy results of allogeneic switchable CAR (allo-RevCAR) T cells against a B-ALL Nalm6-CD20 tumor. Evaluation of tumor control and tumor regression capacity of allo-RevCAR T cells in Nalm6-CD20-Fluc xenograft mice. Mice received 5·10⁵ Nalm6-CD20-Fluc cells 7 days prior to transplantation of 15·10⁶ allo-RevCART cells (total). Therapy was initiated one day before mice received T cells with a bolus injection of 0.2 µg/g or 0.02 µg/g anti-CD19/CD20 targeting module (R-TM19/20) intravenously, respectively. Mice which received allo-RevCAR T cells and targeting module were rechallenged with tumor cells at day 17 (1·10⁶ cells), day 28 (2·10⁶ cells) and day 37 (5·10⁶ cells). Normalized radiance for bioluminescent imaging (BLI) is ranging from 5·10⁴ to 1·10⁷ [p/sec/cm²sr].

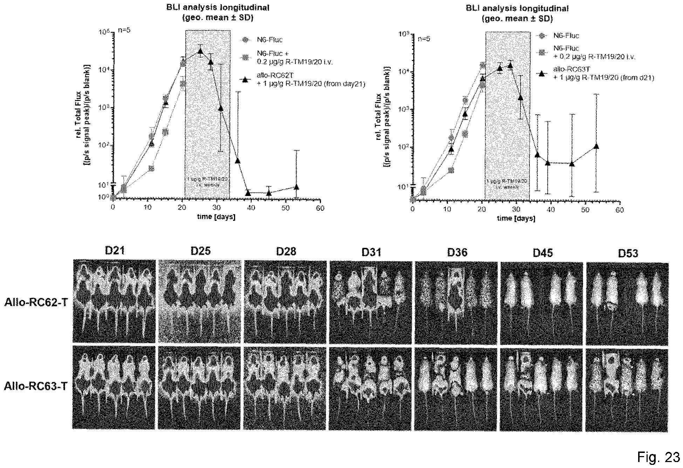

**Fig. 23** shows in vivo efficacy results of allogeneic switchable CAR (allo-RevCAR) T cells against high burden B-ALL Nalm6-CD20 tumor in late-stage mice. Evaluation of tumor control and tumor regression capacity of allogeneic RC62T cells and RC63T cells in late-stage high tumor burden Nalm6-CD20-Fluc xenograft model. Mice received 5·10⁵ Nalm6-CD20-Fluc cells 7 days prior to transplantation of 15·10⁶ allo-RevCAR T cells (total). Therapy was initiated at day 21 after tumor application with intravenous bolus injections of anti-CD19/CD20 targeting module (R-TM19/20) in doses of 1.0 µg/g (day 21, day 28 and day 35) and 0.2 µg/g (day 44). Normalized radiance for BLI is ranging from 5·10⁴ to 1·10⁷ [p/sec/cm²sr].

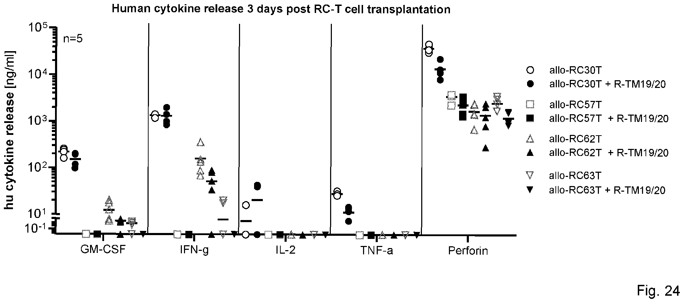

**Fig. 24** shows cytokine release of human allogeneic switchable CAR (allo-RevCAR) T cells in mice. Quantification of human cytokines (GM-CSF, IFN-γ, IL-2, TNF-α and Perforin) from peripheral blood of Nalm6-CD20 xenograft mice three days after allo-RevCAR T transplantation and initiation of therapy with anti-CD19/CD20 targeting module (R-TM19/20). (MACSPlex Cytotoxic T/NK Cell kit from Miltenyi, Germany).

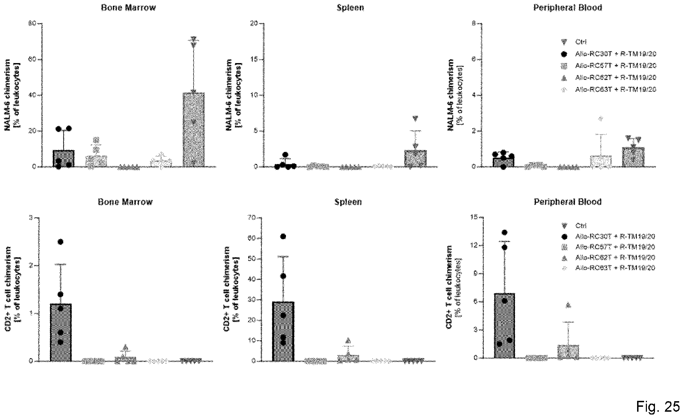

**Fig. 25** shows tumor and T cell chimerism determined at individual end points via flow cytometry. Mice received 5·10⁵ Nalm6-CD20-Fluc cells 7 days prior to transplantation of 15·10⁶ allogeneic switchable CAR (allo-RevCAR) T cells (total). Therapy was initiated one day before mice received allo-RevCAR T cells with a bolus injection of 0.2 µg/g or 0.02 µg/g anti-CD19/CD20 targeting module (R-TM19/20) intravenously, respectively. Mice which received allo-RevCAR T and targeting module were rechallenged with tumor cells at day 17 (1·10⁶ cells), day 28 (2·10⁶ cells) and day 37 (5·10⁶ cells).

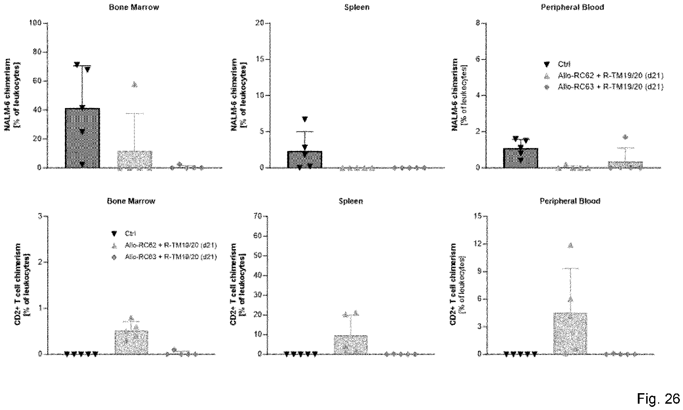

**Fig. 26** shows tumor and T cell chimerism determined at individual end points via flow cytometry in high tumor burden mice. Mice received 5·10⁵ Nalm6-CD20-Fluc cells 7 days prior to transplantation of 15·10⁶ allogeneic switchable CAR (allo-RevCAR) T cells (total). Therapy was initiated at day 21 after tumor application with intravenous bolus injections of anti-CD19/CD20 targeting module (R-TM 19/20) in doses of 1.0 µg/g (day 21, day 28 and day 35) and 0.2 µg/g (day 44).

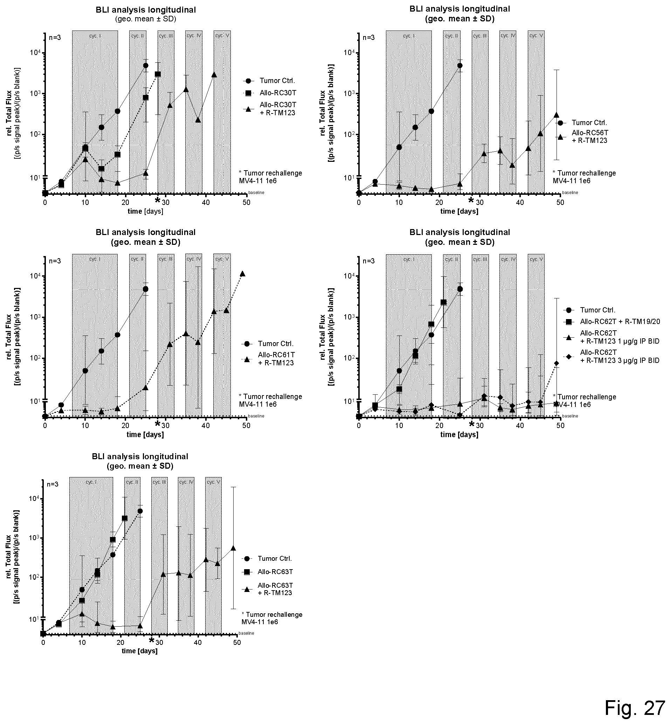

**Fig. 27** shows in vivo efficacy of allogeneic switchable CAR (allo-RevCAR) T cells against an AML MV4-11 tumor. Evaluation of tumor control and tumor regression capacity of allo-RevCAR T cells against MV4-11 Fluc in NSG mice. NSG mice received 5·10⁵ MV4-11-Fluc cells intravenously 7 days prior to transplantation of 15·10⁶ allo-RevCAR T cells (total). Therapy was initiated parallel to allo-RevCAR T cell transplantation with 1 µg/g anti-CD123 targeting module (R-TM123) intraperitoneal twice a day, in cycle of five consecutive days and two days break in between. Mice which received allo-RevCAR T cells and targeting module were rechallenged once with tumor cells at day 28 (1·10⁶ cells). Normalized radiance for BLI is ranging from 5·10⁴ to 1·10⁷ [p/sec/cm²sr].

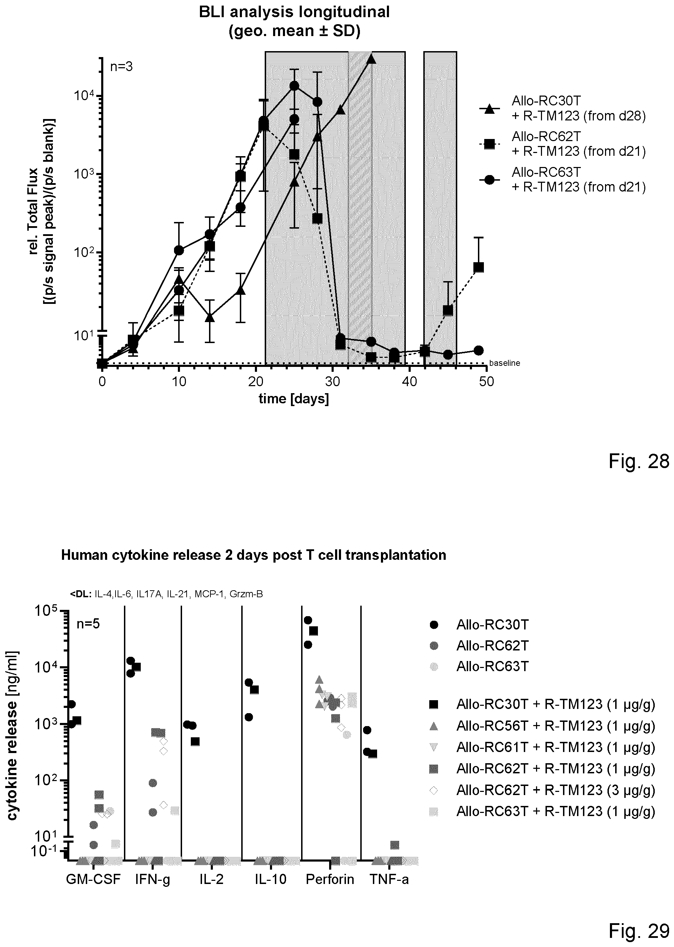

**Fig. 28** shows in vivo efficacy of allogeneic switchable CAR (allo-RevCAR) T cells against an AML MV4-11 tumor in high tumor burden mice. Evaluation of tumor control and tumor regression capacity of allo-RevCAR T against MV4-11 Fluc high tumor burden in NSG mice. NSG mice received 5·10⁵ MV4-11-Fluc cells intravenous 7 days prior to transplantation of 15·10⁶ allo-RevCAR T cells (total). Therapy was initiated at day 21 (for groups treated with allo-RevCAR T cells according to the invention: RC62T and RC63T) and day 28 (from the group treated with RC30T cells as reference) with 1 µg/g anti-CD123 targeting module (R-TM123) intraperitoneal twice a day for 12 consecutive days. For groups treated with RC62T and RC63T cells two more therapy cycles (five days each) with 1 µg/g targeting module intraperitoneal bolus twice a day were applied, first started from day 35 and the second started at day 42. Normalized radiance for BLI is ranging from 5·10⁴ to 1·10⁷ [p/sec/cm²sr].
**Fig. 29** shows cytokine release of human allogeneic switchable CAR (allo-RevCAR) T cells in mice. Quantification of human cytokines (GM-CSF, IFN-γ, IL-2, IL-10, TNF-α and Perforin) from peripheral blood of MV4-11 Fluc engrafted NSG mice two days after allo-RevCAR T cell transplantation and initiation of therapy with anti-CD123 targeting module (R-TM123). Cytokines IL-4, IL-6, IL-17A, IL-21, MCP-1 und Granzyme B were below the detection limit (MACSPlex Cytotoxic T/NK Cell kit from Miltenyi, Germany).

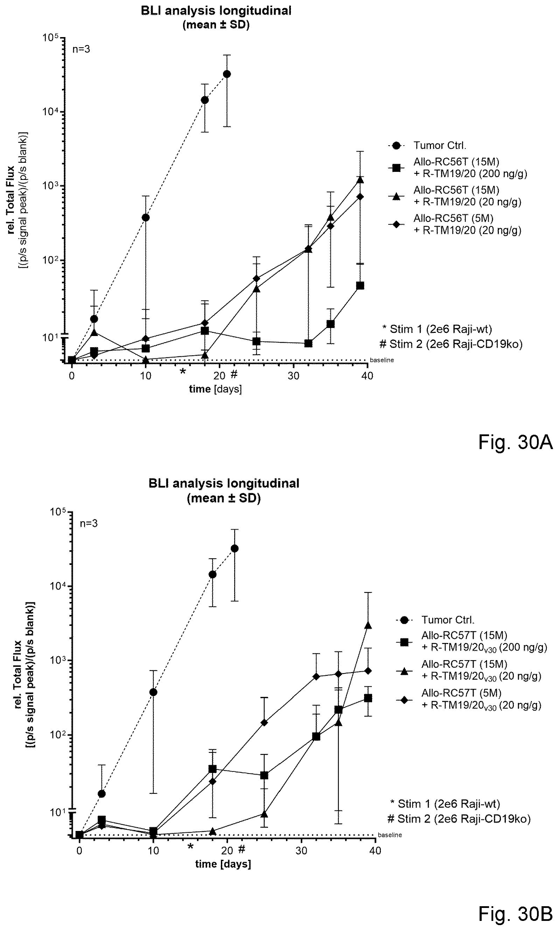

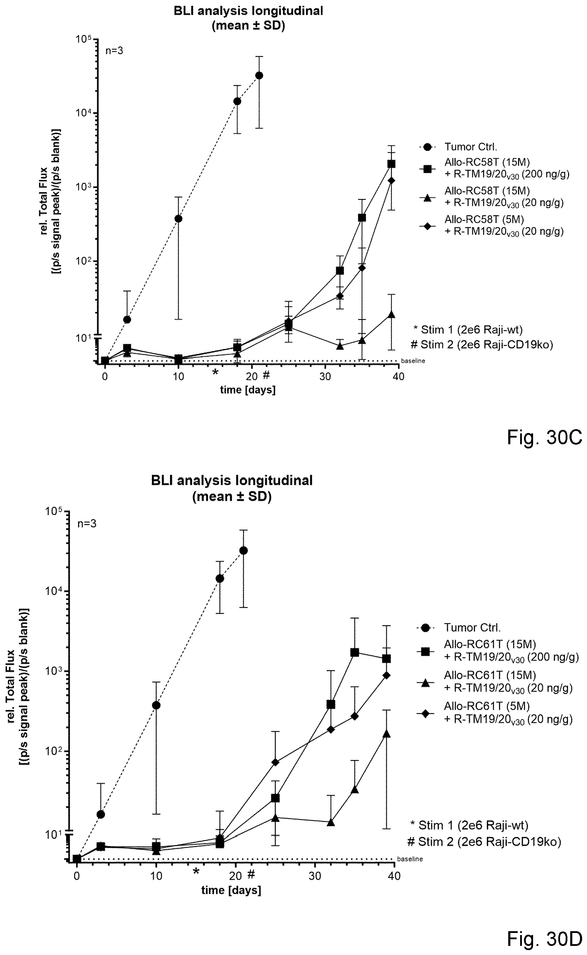

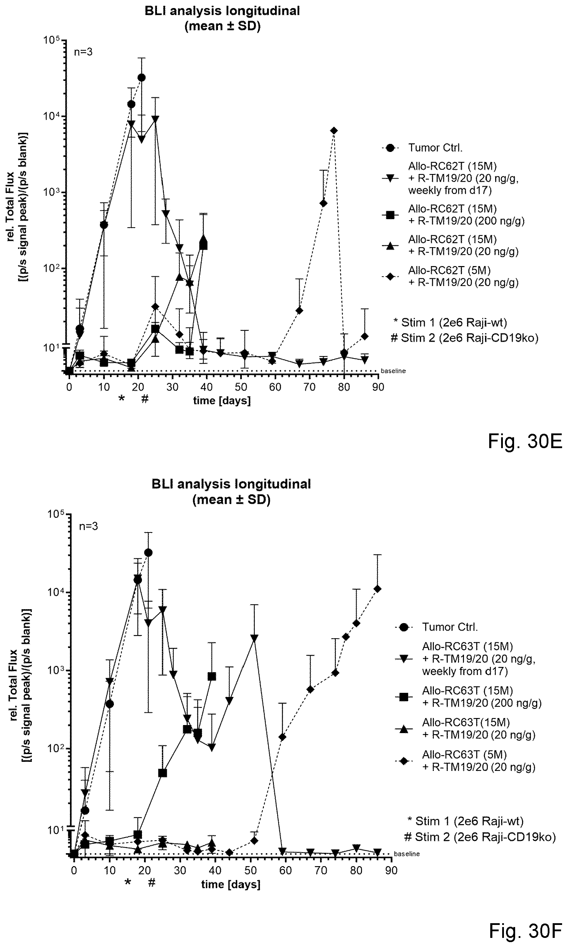

**Fig. 30A to 30F** show in vivo efficacy of allogeneic switchable CAR (allo-RevCAR) T cells against Raji lymphoma. Evaluation of tumor control and tumor regression capacity of allo-RevCAR T cells against Raji-Fluc in NSG mice. NSG mice received 5·10⁵ Raji-Fluc cells intravenously 7 days prior to transplantation of 15·10⁶ or 5·10⁶ allo-RevCAR T cells (total). Therapy was initiated 3 days prior to T cell transplantation with 0.2 µg/g or 0.02 µg/g anti-CD19/CD20 targeting module (R-TM19/20) intravenously and biweekly repeated. Mice which received allo-RevCAR T cells and targeting module were rechallenged once with 2·10⁶ Raji-Fluc tumor cells at day 15 and 2·10⁶ Raji CD19KO tumor cells at days 22. For groups of allo-RevCAR T cells according to the invention: allo-RC62T and allo-RC63T, which initially received no targeting module, therapy started at day 17 with 0.02 µg/g targeting module intravenously and was repeated weekly, here no tumor rechallenge was applied.

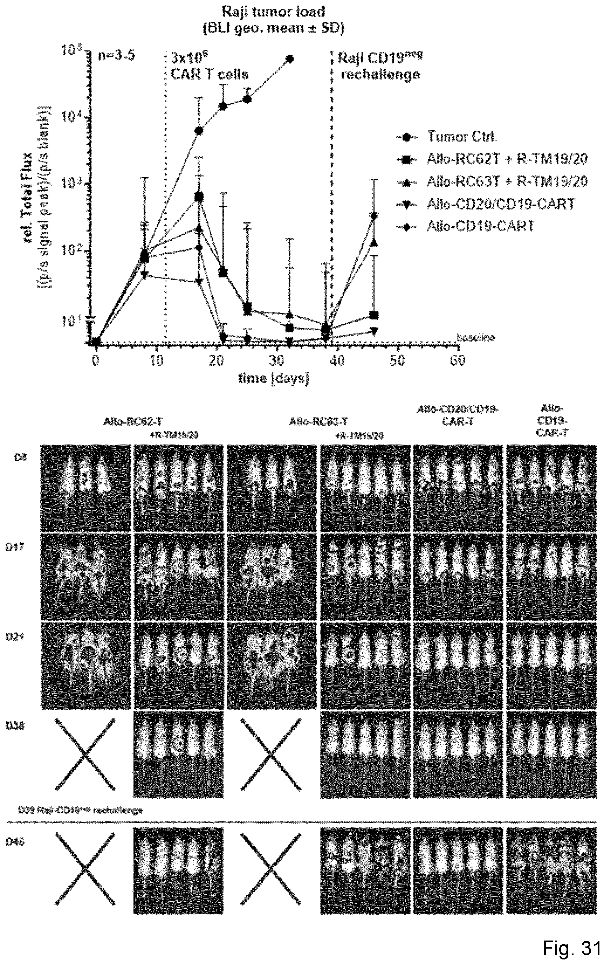

**Fig. 31** shows in vivo efficacy of allogeneic switchable CAR (allo-RevCAR) T cells against B-cell lymphoma. Evaluation of tumor control and tumor regression capacity of allo-RevCAR T cells against Raji-Fluc in NSG mice. NSG mice were engrafted with 5·10⁵ Raji-Fluc cells intravenously 11 days prior to receiving 3·10⁶ CAR T cells intravenously. One day before allo-RevCAR T cells got transplanted, therapy with 0.05 µg/g anti-CD19/CD20 targeting module (R-TM19/20) intravenously started and was repeated weekly. All targeting module treated animals got rechallenged with 2·10⁶ CD19neg Raji cells at day 39. Normalized radiance for BLI is ranging from 5·10⁴ to 1·10⁷ [p/sec/cm²/sr].

### Switchable CAR T cells according to the invention

The immune cells can be genetically engineered to express switchable CARs. For the genetical engineering to express switchable CARs, a polynucleotide vector encoding the switchable CAR and all elements to ensure its expression in the genetically engineered immune cell is transferred into the immune cell. In particular, the switchable CAR comprises a human signal peptide, tag, ECD (extracellular domain), TMD (transmembrane domain), ICD (intracellular domain).

The transfer of the vector can be performed by electroporation or transfection of nucleic acids or the help of viral vector systems like adeno-, adeno-associated, retro-, foamy- or lentiviral viral gene transfer.

The lentiviral gene transfer is applied for stable expression of switchable CARs in immune cells by first constructing a lentiviral vector encoding for a selected switchable CAR. The lentiviral vector is pLVX-EF1alpha UniCAR 28/ζ (Clontech, Takara Bio Group), in which the lentiviral parts of the vector are derived from the human immunodeficiency virus (HIV) and the MSC/IRES/ZxGreenl portion was replaced by the switchable CAR construct.

The lentiviral particles are produced by transient transfection of human embryonal kidney (HEK) 293T (ACC 635) cells with the switchable CAR encoding lentiviral vector plasmid and co-transfection with a group specific antigen (gag) and Polymerase (pol) encoding plasmid (psPAX2) plus a plasmid encoding for an envelope (pMD2.G). After transfection, the packaging plasmid expresses Gag and Pol protein of HIV-1. The plasmid MD2.G encodes the glycoprotein of the vesicular stomatitis virus (VSV-G). VSV-G protein is used to lentiviral vectors to transduce a broad range of mammalian cells. Various envelopes from different virus species can be utilized for this purpose. Lentiviral vectors can successfully pseudotype with the envelope glycoproteins (Env) of amphotropic murine leukemia virus (MLV) or the G protein of vesicular stomatitis virus (VSV-G), a modified envelope of the prototypic foamy virus (PFV) or chimeric envelope glycoprotein variants derived from gibbon ape leukemia virus (GaLV) and MLV.

Supernatants from transfected HEK293T cells are harvested 24 h to 96 h after transfection and virus particles are concentrated from the supernatant by ultracentrifugation or other methods. For lentiviral transduction of immune cells, *peripheral blood mononuclear cells* (PBMC) or isolated T cells are activated with mab specific for the CD3 complex, e.g., clone OKT3 or UCHT1, either given in solution or coated to plastic cell culture dishes or magnetic beads or a biodegradable polymer matrix. Activation of PBMC or isolated T cells is further enhanced by stimulating costimulatory pathways with mabs or ligands specific for CD27, CD28, CD134 or CD137 either alone or in combinations coated to plastic cell culture dishes or magnetic beads or a biodegradable polymer matrix and the supply with exogenous recombinant cytokines like interleukin (IL)-2, IL-7, IL-12, IL-15 and IL-21. Concentrated or non-concentrated virus particles are added to PBMC or T cell cultures 24 h to 96 h after initial administration of activating CD3 specific antibodies and/or antibodies specific for costimulatory receptors CD27, CD28, CD134 or CD137 and/or recombinant cytokines as single or multiple doses. T cell electroporation, transduction and expansion may be performed in open cell culture systems by manual handling or in closed partially or fully automated systems.

Stable transduction of T cells may be determined by flow cytometry after staining with tag containing molecules for surface expression of switchable CARs or mabs directed against the tag or a fourth domain of switchable CARs from day 3 onwards after the final administration of virus supernatant. Switchable CAR transduced T cells can be propagated *in vitro* by culturing them under the supply of recombinant cytokines and activating anti-CD3 mabs.

In case the switchable CAR harbors a tag or the optional fourth domain, a peptide sequence forming a linear epitope for a mab, immune cells genetically modified to express switchable CARs can be specifically propagated *in vitro* by coating a mab or antibody fragments thereof binding to the fourth domain to the surface of culture dishes or to beads of any kind or a biodegradable polymer matrix, which are added to the cell culture at a defined ratio. The binding of surface-coated mabs to the switchable CAR peptide domain induces cross-linkage of cell-surface expressed switchable CARs and formation of an immune synapse, which leads to the activation of signal pathways specifically triggered by the signal domain of the switchable CAR. Depending on the signal pathways induced, this may lead to enhance proliferation and sustained resistance against activation-induced cell death of the switchable CAR-carrying immune cells and therefore enrichment of switchable CAR genetically modified immune cells in a mixed population.

The tag or the optional fourth domain, a peptide sequence forming a linear epitope for a mab, can be further utilized to enrich and purify switchable CAR-expressing immune cells from mixed populations. Enrichment and purification are performed with the help of a mab or antibody fragment thereof binding to the fourth domain to either mark switchable CAR-expressing cells for cell sorting or to transiently link the switchable CAR expressing immune cell to small particles, which can be utilized for cell isolation. In one aspect, switchable CAR-engrafted immune cells are incubated with the mab recognizing the fourth domain. Next, magnetic beads are added, which are conjugated with antibodies or fragments thereof directed against the species- and isotype-specific heavy and light chains of the mab binding to the optional fourth domain. Thus, switchable CAR-expressing immune cells and magnetic beads are linked and are trapped and separated from other immune cells in a magnetic field.

**Tab. 1 Switchable CARs as used in examples**

| Switchable CAR code | SEQ ID No. of switchable CAR | SEQ ID No. of switchable CAR including a leader peptide |
|---|---|---|
| RC30 (reference) | - | 52 |
| RC48 | 53 | 83 |
| RC49 | 54 | 84 |
| RC50 | 55 | 85 |
| RC51 | 56 | 86 |
| RC52 | 57 | 87 |
| RC53 | 58 | 88 |
| RC54 | 59 | 89 |
| RC55 | 60 | 90 |
| RC56 | 61 | 91 |
| RC57 | 62 | 92 |
| RC58 | 63 | 93 |
| RC59 | 64 | 94 |
| RC60 | 65 | 95 |
| RC61 | 66 | 96 |
| RC62 | 67 | 97 |
| RC63 | 68 | 98 |
| RC64 | 69 | 99 |
| RC65 | 70 | 100 |
| RC66 | 71 | 101 |
| RC67 | 72 | 102 |
| RC68 | 73 | 103 |
| RC69 | 74 | 104 |
| RC70 | 75 | 105 |
| RC71 | 76 | 106 |
| RC72 | 77 | 107 |
| RC73 | 78 | 108 |
| RC74 | 79 | 109 |
| RC75 | 80 | 110 |
| RC76 | 81 | 111 |
| RC77 | 82 | 112 |

### Design of targeting modules

The targeting module R-TM123 is a soluble, recombinant fusion protein comprising two antibody-derived binding domains. One selectively binds to the target antigen CD123, the other recognizes a tag presented on the switchable CAR expressing cells (epitope E5B9 from the human La protein). Thus, R-TM123 functions as a bridging module between switchable CAR-T cell and a CD123-expressing target cancer cell. The targeting module further comprises an 8x-histidine tag for detection and purification purposes at the C-terminus.

The targeting module R-TM19/20 is a soluble, recombinant fusion protein comprising three antibody-derived binding domains. One selectively binds to the target antigen CD19, the second selectively binds to the target antigen CD20, the third recognizes a tag presented on the switchable CAR expressing cells (epitope E5B9 from the human La protein). Thus, R-TM19/20 functions as a bridging module between switchable CAR-T cell and a target cancer cell expressing CD19 and/or CD20. The targeting module further comprises an Fc tag for half-life extension, detection, and purification purposes at the C-terminus of its two heavy chains. R-TM19/20 comprises two antibody derived heavy chains and one light chain.

### Characterization of the switchable CAR

T cells transduced to express various switchable CARs, in particular RevCARs, were analyzed for CAR expression with a fluorochrome-labeled anti-tag antibody or GFP as a surrogate marker **(****Fig. 1****).** The switchable CAR-T cells were functionally characterized in vitro **(****Fig. 2 to 9**, 11 to 15 and 19 to 21) and in vivo in immunodeficient mice in conjunction with R-TM19/20 against CD19- and/or CD20-expressing cell lines (i.e. Nalm6, Nalm6-CD20, Raji, CD19 knockout Raji, OCI-AML3-CD19 or OCI-AML3-CD20) **(****Fig. 22** **to26, 30 and 31.** The switchable CAR-T cells were further functionally characterized in conjunction with anti-CD123 targeting module (R-TM123) against CD123-expressing acute myeloid leukemia cell line MV4-11 in vitro **(****Fig. 10****,** **16** **and** **18****)** and in vivo in immunodeficient mice **(****Fig. 27-29****).**

Target cells used in short-term in vitro assay were stained with efluor prior setup and used at indicated effector to target cell (e:t) ratios. Target cells were quantified by flow cytometry and lysis was calculated normalizing the cell count of each sample to a control sample where only tumor cells were plated. Data were fitted with a four-parameter model with a variable slope for sigmoidal curves. In in vitro cytotoxicity assays, efluor stained target cells and switchable CAR T cells were co-cultured at various targeting module concentrations at fixed e:t ratio. The calculated EC₅₀ value can be interpreted as a representative value for the targeting module potency against these tumor cells. In serial killing assays, efluor stained target cells and switchable CAR T cells were co-cultured at various e:t ratios and EC₅₀ value is the e:t ratio at which half-maximal lysis occurs and represents the capacity of switchable CAR T cell products to kill multiple target cells.

Long-term in vitro restimulation co-culture assays were carried out with mcherry positive target cells which were re-added at indicated time points. Mcherry positive target cell counts were determined by fluorescence microscopy (Incucyte S3, Sartorius).

In vivo studies were carried out in immunodeficient mice, which were transplanted with luciferase positive tumor cells and allogeneic switchable CAR T cells at indicated cell numbers at indicated timepoints. Tumor growth was followed by bioluminescence imaging.

Switchable CAR T cell chimerism was monitored by flow cytometry. Cytokine secretion by switchable CAR T cells was quantified using the MACSPlex Cytotoxic T/NK Cell kit (Miltenyi, Germany).

### Cited non-patent literature

Cartellieri M, Bachmann M, Feldmann A, Bippes C, Stamova S, Wehner R, Temme A, Schmitz M (2010) Chimeric Antigen Receptor-Engineered T Cells for Immunotherapy of Cancer J. Biomed. Biotechnol. Article ID 956304, doi: 10.1155/2010/956304.
Cartellieri M, Feldmann A, Koristka S, Arndt C, Loff S, Ehninger A, von Bonin M, Bejestani EP, Ehninger G, Bachmann MP (2016) Switching CAR T cells on and off: a novel modular platform for retargeting of T cells to AML blasts. Blood cancer J. 6 (8), e458.
Fedorov VD, Themeli M, Sadelain M (2013) PD-1- and CTLA-4-based inhibitory chimeric antigen receptors (iCARs) divert off-target immunotherapy responses. Sci. Transl. Med. 5 (215), 215ra172.
Finney HM, Akbar AN, Lawson AD (2004) Activation of resting human primary T cells with chimeric receptors: costimulation from CD28, inducible costimulator, CD134, and CD137 in series with signals from the TCR zeta chain. J. Immunol. 172 (1), 104-113.
Gong MC, Latouche JB, Krause A, Heston WDW, Bander NH, Sadelain M (1999) Cancer patient T cells genetically targeted to prostate-specific membrane antigen specifically lyse prostate cancer cells and release cytokines in response to prostate-specific membrane antigen. Neoplasia. 1 (2), 123-127.
Guedan S, Posey AD, Shaw C, Wing A, Da T, Patel PR, McGettigan SE, Casado-Medrano V, Kawalekar OU, Uribe-Herranz M, Song D, Melenhorst JJ, Lacey SF, Scholler J, Keith B, Young RM, June CH (2018) Enhancing CAR T cell persistence through ICOS and 4-1BB costimulation. JCI Insight. 3 (1), 96976.
Guedan S, Madar A, Casado-Medrano V, Shaw CE, Wing A, Liu F, Young RM, June CH, Posey AD (2020) Single residue in CD28-costimulated CAR T cells limits long-term persistence and antitumor durability. J Clin Invest. 133215.
Guest RD, Hawkins RE, Kirillova N, Cheadle EJ, Arnold J, O'Neill A, Irlam J, Chester KA, Kemshead JT, Shaw DM, Embleton MJ, Stern PL, Gilham DE (2005) The role of extracellular spacer regions in the optimal design of chimeric immune receptors: evaluation of four different scFvs and antigens. J Immunother. 28(3), 203-211.
Hombach A, Sent D, Schneider C, Heuser C, Koch D, Pohl C, Seliger B, Abken H (2001) T-cell activation by recombinant receptors: CD28 costimulation is required for interleukin 2 secretion and receptor-mediated T-cell proliferation but does not affect receptor-mediated target cell lysis. Cancer Res. 61 (5), 1976-1982.
Jame SE, Greenberg PD, Jensen MC, Lin Y, Wang J, Till BG, Raubitschek AA, Forman SJ, Press OW (2008) Antigen sensitivity of CD22-specific chimeric TCR is modulated by target epitope distance from the cell membrane. J Immunol 180, 7028-7038.
Kagoya Y, Tanaka S, Guo T, Anczurowski M, Wang CH, Saso K, Butler MO, Minden MD, Hirano N (2018) A novel chimeric antigen receptor containing a JAK-STAT signaling domain mediates superior antitumor effects. Nat Med. 24 (3), 352-359.
Morgan RA, Yang JC, Kitano M, Dudley ME, Laurencot CM, Rosenberg SA (2010) Case Report of a Serious Adverse Event Following the Administration of T Cells Transduced With a Chimeric Antigen Receptor Recognizing ERBB2. Mol. Ther. 18, 843-851.
Patel SD, Moskalenko M, Smith D, Maske B, Finer MH, McArthur JG (1999) Impact of chimeric immune receptor extracellular protein domains on T cell function. Gene Therap. 6, 412-419.
Reinke AW, Grant RA, Keating AE (2010) A Synthetic Coiled-Coil Interactome Provides Heterospecific Modules for Molecular Engineering. JACS 132, 6025-6031.
Sotillo E, Barrett DM, Black K., Bagashev A, Oldridge D, Wu G, Sussman R, Lanauze C, Ruella M, Gazzara MR, Martinez NM, Harrington CT, Chung EY, Perazzelli J, Hofmann TJ, Maude SL, Raman P, Barrera A, Gill S, Lacey SF, Melenhorst JJ, Allman D, Jacoby E, Fry T, Mackall C, Barash Y, Lynch KW, Maris JM, Grupp SA, Thomas-Tikhonenko A (2015) Convergence of Acquired Mutations and Alternative Splicing of CD19 Enables Resistance to CART-19 Immunotherapy. Cancer Discov. 5, 1282-1295.
Titov A, Petukhov A, Staliarova A, Motorin D, Bulatov E, Shuvalov O, Soond SM, Piacentini M, Melino G, Zaritskey A, Barlev NA (2018) The biological basis and clinical symptoms of CAR-T therapy-associated toxicites. Cell Death Dis 9, 897.
Töpfer K, Cartellieri M, Michen S, Wiedemuth R, Müller N, Lindemann D, Bachmann M, Füssel M, Schackert G, Temme A (2015) DAP12-based activating chimeric antigen receptor for NK cell tumor immunotherapy. J. Immunol. 194 (7), 3201-3212.
Weber EW, Parker KR, Sotillo E, Lynn RC, Anbunathan H, Lattin J, Good Z, Belk JA, Daniel B, Klysz D, Malipatlolla M, Xu P, Bashti M, Heitzeneder S, Labanieh L, Vandris P, Majzner RG, Qi Y, Sandor K, Chen LC, Prabhu S, Gentles AJ, Wandless TJ, Satpathy AT, Chang HY, Mackall CL (2021) Transient rest restores functionality in exhausted CAR-T cells through epigenetic remodeling. Science 372 (6537), eaba1786.
Zhang T, Lemoi BA, Sentman CL (2005) Chimeric NK-receptor-bearing T cells mediate antitumor immunotherapy. Blood. 106 (5), 1544-1551.

## Claims

1. A switchable chimeric antigen receptor comprising
a. a tag or tag-binding domain,
b. an extracellular hinge and transmembrane domain comprising an extracellular hinge and a transmembrane domain of CD4, CD8α, CD28, ICOS (CD278), IgG1, IgG2, IgG4 or mutants and combinations thereof,
wherein the extracellular hinge and transmembrane domain is in the range of 32 to 159 amino acids, and
c. an intracellular signaling domain that comprises at least two signal transduction domains independently selected from the group comprising a cytoplasmic region of CD3, CD27, CD28, OX40 (CD134), 4-1BB (CD137), ICOS (CD278), DAP10, DAP12, PD-1, CTLA-4, IL-2 receptor, IL-7 receptor, IL-15 receptor or IL-21 receptor and mutants thereof.

2. The switchable chimeric antigen receptor according to claim 1, wherein the tag is a peptide epitope tag.

3. The switchable chimeric antigen receptor according to claim 2, wherein the peptide epitope tag is a myc-tag, a His-tag, a peptide sequence from yeast transcription factor GCN4, a leucine zipper sequence or a peptide sequence from a human protein.

4. The switchable chimeric antigen receptor according to claim 1, wherein the tag-binding domain is an antibody or antigen-binding fragment, a protein or a peptide binding to a myc-tag, a His-tag, a peptide sequence from yeast transcription factor GCN4, a leucine zipper sequence or a peptide sequence from a human protein.

5. The switchable chimeric antigen receptor according to one of the claims 1 to 4, wherein the extracellular hinge and transmembrane domain comprises an extracellular hinge and a transmembrane domain of CD8α, CD28 or IgG4 and mutants thereof.

6. The switchable chimeric antigen receptor according to one of the claims 1 to 5, wherein the intracellular signaling domain comprises at least a cytoplasmic region of CD3ζ, 4-1BB, CD28 or mutants thereof.

7. The switchable chimeric antigen receptor according to one of the claims 1 to 6, wherein the switchable chimeric antigen receptor is a reversible chimeric antigen receptor comprising a tag.

8. The switchable chimeric antigen receptor according to one of the claims 1 to 7 comprising a signal peptide selected from human CD8α, CSF2Rα, CD3ζ, IL-2, lysozyme C, a heavy chain of an antibody, a light chain of an antibody or a part of a light chain of an antibody.

9. The switchable chimeric antigen receptor according to one of the claims 1 to 8 comprising a sequence according to SEQ ID No. 53 to SEQ ID No. 82.

10. A nucleic acid, vector or cell comprising a nucleotide sequence encoding the switchable CAR according to one of the claims 1 to 9.

11. The vector according to claim 10, wherein the vector is selected from the group comprising a DNA vector, an RNA vector, a plasmid, a lentiviral vector, retroviral vector, adenoviral vector and an adeno-associated viral vector.

12. The vector of claim 11, further comprising a promoter, wherein the promoter is selected from the group comprising an EF-1 promoter, a CMV IE gene promoter, an EF-1α promoter, a ubiquitin C promoter, or a phosphoglycerate kinase (PGK) promoter.

13. The cell according to claim 10, wherein the cell is an immune effector cell selected from the group comprising a cytotoxic T lymphocyte, regulatory T cell, Natural Killer cell, and macrophage.

14. A pharmaceutical composition comprising a cell comprising a nucleotide sequence encoding the switchable CAR according to one of the claims 1 to 9 and a pharmaceutically acceptable thinner or carrier.

15. A kit comprising
a) a nucleic acid, vector and/or cell comprising a nucleotide sequence encoding the switchable CAR according to one of the claims 1 to 9, and
b) a targeting module comprising at least one target cell-binding domain capable of binding a target antigen and a tag or tag-binding domain and/or at least one nucleic acid, vector and/or cell encoding a targeting module,
wherein the tag-binding domain of the targeting module binds to the tag of the switchable chimeric antigen receptor or the tag of the targeting module binds to the tag-binding domain of the switchable chimeric antigen receptor.

16. The kit according to claim 15, wherein the target cell-binding domain of the targeting module is an antibody, antigen-binding fragment, a protein, a peptide or a low molecular weight organic ligand that binds to a surface antigen selected from the group comprising CD2, CD3, CD4, CD5, CD7, CD8, CD10, CD15, CD19, CD20, CD22, CD23, CD25, CD30, CD33, CD38, CD44, CD44v6, CD52, CD66a, CD66b, CD66c, CD66d, CD66e, CD66f, CD79a, CD79b, CD90, CD99, CD123, CD133, CD135, CD150, CD181, CD182, CD184, CD223, CD229, CD269, CD273, CD274, CD276, CD279, CD319, CD366, CD371, cytokine receptors, CXCR4, c-Met, mesothelin, a member of the epidermal growth factor receptor family or a mutant thereof, a member of the tumor necrosis factor receptor superfamily, a claudin, an ephrin, an ephrin receptor, a fucosyl transferase, a prostate specific antigen, an embryonic antigen, a member of the vascular endothelia growth factor family, epithelial cell adhesion molecule, alpha-fetoprotein, a member of the intercellular adhesion molecule family, a C-type lectin, an integrin, a member of the mucin protein family, a follicle-stimulating hormone receptor, a high molecular weight-melanoma associated antigen, a folate binding protein, a folate receptor, a somatostatin receptor, a ligand of the NKG2D receptor, a member of the epithelia glycoprotein family, a disialoganglioside, a glypican, a G protein-coupled receptor, a human papillomavirus protein, cancer/testis antigen, fibroblast activation protein, a member of the carbonic anhydrase family, a member of the carbohydrate antigen family, a Notch ligand, melanoma-associated chondroitin sulfate proteoglycan, glycoprotein A33, guanylate cyclase 2C and tumor-specific glycan.

17. The kit according to claim 15 or 16, wherein the length of the targeting module is in the range of 20 to 1600 amino acids, preferably 200 to 1450 amino acids.

18. The kit according to one of the claims 15 to 17, wherein the cell comprising a nucleotide sequence encoding a reversible chimeric antigen receptor and/or the targeting module are in the form of a pharmaceutical composition.

19. The kit according to one of the claims 15 to 18, wherein the kit further comprises at least one further targeting module or at least one further nucleic acid, vector or cell encoding a further targeting module,
wherein the at least one further targeting module comprises at least one target cell-binding domain and a tag-binding domain or a tag,
wherein the at least one target cell-binding domain of the further targeting module is an antibody, antigen-binding fragment, a protein, a peptide or a low molecular weight organic ligand that binds to a surface antigen selected from the group comprising CD2, CD3, CD4, CD5, CD7, CD8, CD10, CD15, CD19, CD20, CD22, CD23, CD25, CD30, CD33, CD38, CD44, CD44v6, CD52, CD66a, CD66b, CD66c, CD66d, CD66e, CD66f, CD79a, CD79b, CD90, CD99, CD123, CD133, CD135, CD150, CD181, CD182, CD184, CD223, CD229, CD269, CD273, CD274, CD276, CD279, CD319, CD366, CD371, cytokine receptors, CXCR4, c-Met, mesothelin, a member of the epidermal growth factor receptor family or a mutant thereof, a member of the tumor necrosis factor receptor superfamily, a claudin, an ephrin, an ephrin receptor, a fucosyl transferase, a prostate specific antigen, an embryonic antigen, a member of the vascular endothelia growth factor family, epithelial cell adhesion molecule, alpha-fetoprotein, a member of the intercellular adhesion molecule family, a C-type lectin, an integrin, a member of the mucin protein family, a follicle-stimulating hormone receptor, a high molecular weight-melanoma associated antigen, a folate binding protein, a folate receptor, a somatostatin receptor, a ligand of the NKG2D receptor, a member of the epithelia glycoprotein family, a disialoganglioside, a glypican, a G protein-coupled receptor, a human papillomavirus protein, cancer/testis antigen, fibroblast activation protein, a member of the carbonic anhydrase family, a member of the carbohydrate antigen family, a Notch ligand, melanoma-associated chondroitin sulfate proteoglycan, glycoprotein A33, guanylate cyclase 2C and tumor-specific glycan,
wherein the targeting module and the at least one further targeting module comprise different target cell-binding domains, and identical tag-binding domains or tags.

20. The switchable chimeric antigen receptor according to one of the claims 1 to 9, the cell according to claims 10 or 13, the pharmaceutical composition according to claim 14 or the kit according to one of the claims 15 to 19 for use as a medicament.

21. The switchable chimeric antigen receptor according to one of the claims 1 to 9, the cell according to claims 10 or 13, the pharmaceutical composition according to claim 14 or the kit according to one of the claims 15 to 19 for use in the treatment of cancer, infectious disease or autoimmune disease.
